# EUROPÄISCHE PATENTSCHRIFT

(11) **EP 2 318 383 B1**
(45) Veröffentlichungstag und Bekanntmachung des Hinweises auf die Patenterteilung: **03.07.2013**
(21) Anmeldenummer: 09776375.9
(22) Anmeldetag: 13.02.2009
(51) Int. Cl.: C07D 295/14

(54) **VERFAHREN ZUR HERSTELLUNG VON CYCLOALKYL-SUBSTITUIERTEN PIPERAZINVERBINDUNGEN**
PROCESS FOR PREPARING CYCLOALKYL-SUBSTITUTED PIPERAZINE COMPOUNDS
PROCÉDÉ DE FABRICATION DE COMPOSÉS DE PIPÉRAZINE À SUBSTITUTION CYCLOALKYLE

(30) Priorität: 12.08.2008 WO PCT/EP2008/060562
(43) Veröffentlichungstag der Anmeldung: 11.05.2011
(73) Patentinhaber: Boehringer Ingelheim International GmbH, 55216 Ingelheim am Rhein (DE)
(72) Erfinder: PFRENGLE, Waldemar, 55216 Ingelheim Am Rhein (DE); FRANK, Markus, 55216 Ingelheim Am Rhein (DE); PACHUR, Thorsten, 55216 Ingelheim Am Rhein (DE); HUCHLER, Günther, 55216 Ingelheim Am Rhein (DE)
(74) Vertreter: Simon, Elke Anna Maria
(86) Internationale Anmeldenummer: PCT/EP2009/001012
(87) Internationale Veröffentlichungsnummer: WO 2010/017850

(56) Entgegenhaltungen:
- WO-A-2006/021544
- WO-A-2008/022945
- WO-A-2008/145681
- WO-A-2009/021944

## Beschreibung

Die vorliegende Erfindung betrifft ein Verfahren zur Herstellung von Verbindungen der allgemeinen Formel **I** 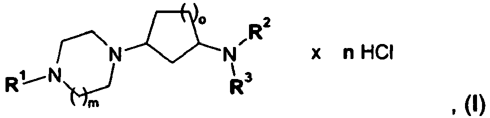 in der m, n, o, **R¹, R²** und **R³** wie nachstehend erwähnt definiert sind, deren Enantiomere und deren Diastereomere, welche zur Herstellung von Verbindungen der allgemeinen Formel **II** 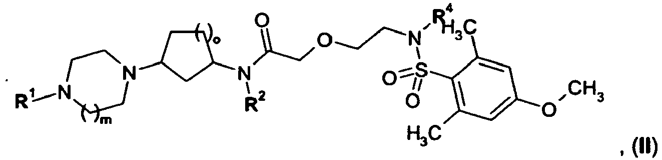 in der m, o, **R¹, R², R³** und **R⁴** wie nachstehend erwähnt definiert sind, besonders geeignet sind. Die Verbindungen der allgemeinen **Formel II** besitzen B1-antagonistische Eigenschaften.

"Im Stand der Technik (WO 2006/021544) wird die Herstellung von ausgewählten Verbindungen der allgemeinen Formel I, in denen o die Ziffer 0 bedeutel (= Cyclobutylderivate), durch Umsetzung von tosylierten Cyclobutylcarbaminsäuren mit Basen wie Pyrrolidin oder Piperazin beschrieben. Die vorliegende Erfindung beschreibt die Herstellung von Verbindungen der allgemeinen Formel J durch Umsetzung der entsprechenden Piperazinderivate mit ungesältigten cyclischen Ketonen bzn:Lactonen."

Ein erster Gegenstand der vorliegenden Erfindung betrifft ein Verfahren zur Herstellung von Verbindungen der allgemeinen Formel I 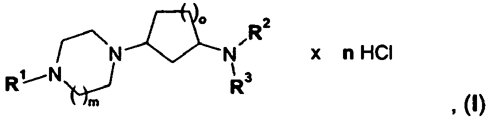 in der
- **m**: eine der Ziffern 1 oder 2,
- **n**: eine der Ziffern 0, 1, 2 oder 3,
- **o**: eine der Ziffern 0, 1, 2 oder 3,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)- oder
(e) C₁₋₄-Alkyl-C(O)-, die mit 1, 2 oder 3 Fluor- oder Chloratomen substituiert sein kann, und
- **R³**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)- oder
(e) C₁₋₄-Alkyl-C(O)-, die mit 1, 2 oder 3 Fluor- oder Chloratomen substituiert sein kann,
deren Enantiomere und deren Diastereomere bedeuten, umfassend die folgenden Schritte:
(a) Addition einer Verbindung der allgemeinen Formel **III** 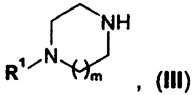 in der **m** und **R¹** wie voranstehend erwähnt definiert sind, an eine Verbindung der allgemeinen Formel **IV** 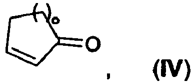 in der **o** wie voranstehend erwähnt definiert ist;
(b) Umsetzung einer unter Schritt (a) erhaltenen Verbindung der allgemeinen Formel **V** 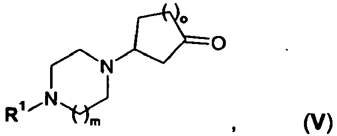 in der **m**, **o** und **R¹** wie voranstehend erwähnt definiert sind, mit Hydroxylamin-Hydrochlorid;
(c) Reduktion eines unter Schritt (b) erhaltenen Oxims der allgemeinen Formel **VI** 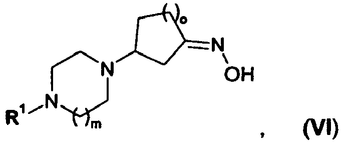 in der **m**, **o** und **R¹** wie voranstehend erwähnt definiert sind, in Gegenwart eines Katalysators;
(d) gegebenenfalls Isolierung einer unter Schritt (c) erhaltenen Verbindung der allgemeinen Formel Ia 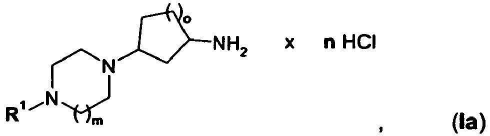 in der **m, n, o** und **R¹** wie voranstehend erwähnt definiert sind;
(e) Kupplung eines unter Schritt (c) oder (d) erhaltenen Amins der allgemeinen Formel **Ia** 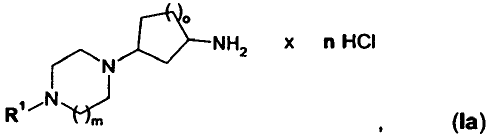 in der **m, n, o** und **R¹** wie voranstehend erwähnt definiert sind, mit einer Verbindung der allgemeinen Formel **VII**

   X-R², (VII)

   in der **R²** wie voranstehend erwähnt definiert ist und **X** eine Abgangsgruppe, beispielsweise ein Halogenatom, eine Tosylat-, Mesylat-, Triflat- oder eine Hydroxysuccinimid-gruppe, darstellt;
(f) gegebenenfalls Isolierung einer unter Schritt (e) erhaltenen Verbindung der allgemeinen Formel **Ib** 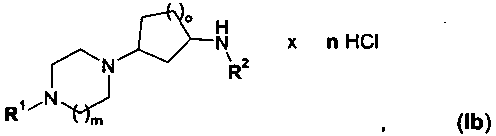 in der m, n, o, **R¹** und **R²** wie voranstehend erwähnt definiert sind;
(g) gegebenenfalls erneute Kupplung einer unter Schritt (e) oder (f) erhaltenen Verbindung der allgemeinen Formel **Ib** 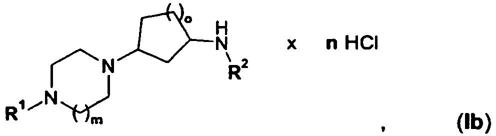 in der **m, n, o, R¹** und **R²** wie voranstehend erwähnt definiert sind, mit einer Verbindung der allgemeinen Formel **VIII**

   X-R³, (VIII)

   in der **R³** wie voranstehend erwähnt definiert ist und **X** eine Abgangsgruppe, beispielsweise ein Halogenatom, eine Tosylat-, Mesylat-, Triflat- oder eine Hydroxysuccinimid-gruppe, darstellt;
(h) gegebenenfalls Isolieren einer unter Schritt (g) erhaltenen Verbindung der allgemeinen Formel **1;**
(i) gegebenenfalls stereoselektive Trennung oder Anreicherung der Stereoisomere einer unter Schritt (c) oder (d) erhaltenen Verbindung der allgemeinen Formel **Ia** oder einer unter Schritt (e) oder (f) erhaltenen Verbindung der allgemeinen Formel **Ib** oder einer unter Schritt (g) oder (h) erhaltenen Verbindung der allgemeinen Formel **I**, durch Co-Kristallisation oder Salzbildung mit anorganischen Säuren oder chiralen Säuren;
(j) gegebenenfalls Isolierung eines oder mehrerer unter Schritt (i) erhaltenen Stereoisomere der allgemeinen Formel **IX** 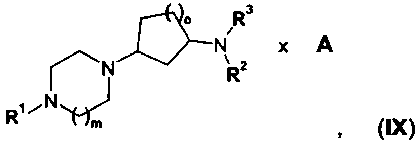 in der **m, n, o, R¹, R²** und **R³** wie voranstehend erwähnt definiert sind und **A** eine oder mehrere chirale Säuren oder ein oder mehrere entsprechende Anionen einer oder mehrerer anorganischer Säuren bedeutet;
(k) Umsetzung einer unter Schritt (i) oder (j) erhaltenen Verbindung der allgemeinen Formel **IX** mit einer Base;
(l) gegebenenfalls Isolieren einer stereoisomeren oder enantiomerenagreicherten Verbindung der allgemeinen Formel I 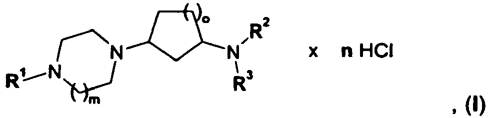 in der **m, n, o, R¹, R²** und **R³** wie voranstehend erwähnt definiert sind; und
(m) gegebenenfalls anschließendes Entfernen einer Aminschutzgruppe in einer so erhaltenen Verbindung der allgemeinen **Formel I,** in der **m, n** und **o** wie voranstehend erwähnt definiert sind und mindestens einer der Reste **R¹, R²** oder **R³** eine Aminschutzgruppe, beispielsweise eine Benzyl-, C₁₋₄-Alkyl-O-C(O)-, Benzyl-O-C(O)-, Acetyl-, Trifluoracetyl- oder Trichloracetylgruppe, trägt, wodurch eine Verbindung der allgemeinen Formel I erhalten wird, in der **m, n** und **o** wie voranstehend erwähnt definiert sind und mindestens einer der Reste **R¹, R²** oder **R³** ein Wasserstoffatom bedeutet; und
(n) gegebenenfalls Reduktion einer so erhaltenen Verbindung der allgemeinen Formel **l,** in der **m, n, o** und **R¹** wie voranstehend erwähnt definiert sind und mindestens einer der Reste **R²** oder **R³** eine C₁₋₄-Alkyl-O-C(O)- oder Benzyl-O-C(O)-Gruppe bedeutet, mit einem Reduktionsmittel, wodurch eine Verbindung der allgemeinen Formel I erhalten wird, in der **m, n, o** und **R¹** wie voranstehend erwähnt definiert sind und mindestens einer der Reste **R²** oder **R³** eine Methylgruppe darstellt.

Das Produkt jeder Stufe kann nach literaturbekannten, geeigneten Methoden, wie z.B. durch Kristallisation, Chromatographie oder Eindampfen zur Trockene, isoliert werden.

Bei der Addition in Schritt (a) werden vorzugsweise 1.0 Äquivalente einer Verbindung der allgemeinen Formel **III** mit 1.0 bis 1.5 Äquivalenten, vorzugsweise 1.0 bis 1.2 Äquivalenten, einer Verbindung der allgemeinen Formel **IV** entweder lösungsmittelfrei oder in einem polaren organischen Lösungsmittel umgesetzt. Als polares organisches Lösungsmittel können Methanol, Ethanol, Propanol, Isopropanol, Aceton, Isopropylacetat oder Ethylacetat oder Mischungen dieser Lösungsmittel verwendet werden. Das Lösungsmittel wird vorzugsweise in einer Menge von 0.2 bis 0.4 Umol eingesetzter Verbindung der allgemeinen Formel **III,** vorzugsweise in einer Menge von 0.25 bis 0.35 Umol eingesetzter Verbindung der allgemeinen Formel **IV,** zugegeben.

Bei der Umsetzung in Schritt (b) werden vorzugsweise 1.0 Äquivalente einer Verbindung der allgemeinen Formel V mit 1.0 bis 1.5 Äquivalenten, vorzugsweise 1.1 bis 1.3 Äquivalenten, Hydroxylamin-Hydrochlorid in einem polaren organischen Lösungsmittel umgesetzt. Als polares organisches Lösungsmittel kann Methanol, Ethanol, Propanol, Isopropanol, Aceton, Isopropylacetat oder Ethylacetat oder Mischungen dieser Lösungsmittel verwendet werden. Das Lösungsmittel wird vorzugsweise in einer Menge von 0.6 bis 1.2 Umol eingesetzter Verbindung der allgemeinen Formel **V,** vorzugsweise in einer Menge von 0.75 bis 1.1 L/mol eingesetzter Verbindung der allgemeinen Formel **V,** zugegeben.

Die Umsetzung in Schritt (b) kann auch in Gegenwart einer anorganischen Base durchgeführt werden. Die Base wird vorzugsweise in einer Menge von 1.0 bis 1.5 Äquivalenten, vorzugsweise 1.1 bis 1.3 Äquivalenten, zugegeben, bezogen auf die Menge an eingesetzter Verbindung der allgemeinen Formel **V.** Verwendet werden können Lithiumcarbonat, Natriumcarbonat, Kaliumcarbonat oder Natriumhydrogencarbonat, wobei Kaliumcarbonat erfindungsgemäß bevorzugt verwendet wird.

Alternativ zur voranstehend unter den Schritten (a) und (b) beschriebenen Synthese kann eine Verbindung der allgemeinen Formel **VI** auch durch Addition einer Verbindung der allgemeinen Formel **III** 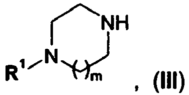 in der m und **R¹** wie voranstehend erwähnt definiert sind, an eine Verbindung der allgemeinen Formel **IVa** 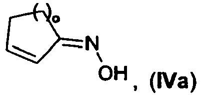 in der **o** wie voranstehend erwähnt definiert ist, hergestellt werden.

Bei der Reduktion in Schritt (c) werden vorzugsweise 1.0 Äquivalente einer Verbindung der allgemeinen Formel **VI** in Wasser oder einem organischen Lösungsmittel in Gegenwart eines Reduktionsmittels und gegebenenfalls in Gegenwart einer Base umgesetzt. Als organisches Lösungsmittel kann Methanol, Ethanol, Propanol, Butanol, Ethylacetat, Toluol, Xylol, Tetrahydrofuran, Methyl-Tetrahydrofuran oder ein Gemisch dieser Lösungsmittel verwendet werden. Das Lösungsmittel wird vorzugsweise in einer Menge von 1.5 bis 2.5 Umol eingesetzter Verbindung der allgemeinen Formel **VI,** vorzugsweise von 1.9 bis 2.1 Umol eingesetzter Verbindung der allgemeinen Formel **VI** verwendet. Die Base wird vorzugsweise in einer Menge von 0.02 bis 0.2 Äquivalenten, vorzugsweise 0.07 bis 0.15 Äquivalenten, zugegeben, jeweils bezogen auf die Menge an eingesetzter Verbindung der allgemeinen Formel **VI.** Als Base verwendet werden können Ammoniak, Triethylamin, Diisopropylethylamin oder Diazabicyclo[5.4.0]undec-7-en (DBU), wobei Ammoniak erfindungsgemäß bevorzugt verwendet wird.

Das Reduktionsmittel kann ausgewählt sein aus der Gruppe bestehend aus Wasserstoff, Wasserstoff/Palladium/Kohle, Wasserstoff/Palladium oder Wasserstoff/Raney-Nickel, Ameisensäure, Formiaten, komplexen Metallhydriden, Natrium/Alkohole, Zink/Essigsäure, Zinn/Salzsäure, wobei bevorzugt Wasserstoff/Palladium/Kohle verwendet werden. Es können 1 bis 3 Äquivalente, bevorzugt 1.5 bis 2.5 Äquivalente, des Reduktionsmittels zugegeben werden, jeweils bezogen auf die Menge eingesetzter Verbindung der allgemeinen Formel **VI.**

Vorteilhafte Bedingungen für die Hydrierung sind Temperaturen von 20 bis 60°C, vorzugsweise 25 bis 35°C, und ein Wasserstoffüberdruck von maximal 5 bar.

Nach dem Abfiltrieren des Katalysators kann das Hydrierungsprodukt durch Abdestillieren des Lösungsmittels aufkonzentriert werden. Nach Aufarbeitung wird eine Verbindung der allgemeinen Formel **Ia** erhalten, in der **n** die Ziffer 0 bedeutet.

Die unter Schritt (d) beschriebene Isolierung einer Verbindung der allgemeinen Formel **Ia** kann in Form des freien Amins erfolgen, wobei **n** die Ziffer 0 darstellt.

Eine so erhaltene Verbindung kann anschließend in einem Lösungsmittel gelöst und durch Zugabe einer entsprechenden Menge Salzsäure in eine Verbindung der allgemeinen Formel **Ia** übergeführt werden, in der **n** eine der Ziffern 1, 2 oder 3, vorzugsweise die Ziffer 3, bedeutet.

Als Lösungsmittel kann dabei Methanol, Ethanol, Propanol, Butanol, Isopropanol, tert-Amylalkohol, Isopropylacetat, Tetrahydrofuran, Methyltetrahydrofuran, Dioxan, Ethylacetat, Dichlormethan, Methylcyclohexan oder Toluol verwendet werden.

Bei der Kupplung in Schritt (e) werden vorzugsweise 1.0 Äquivalente einer Verbindung der allgemeinen Formel **Ia** mit 1.0 bis 1.5 Äquivalenten, vorzugsweise 1.0 bis 1.2 Äquivalenten, einer Verbindung der allgemeinen Formel **VII** in einem Lösungsmittel und in Gegenwart einer Base umgesetzt. Als Lösungsmittel kann Wasser, Methanol, Ethanol, Propanol, Butanol, Isopropanol, tert-Amylalkohol, Aceton, Methylcyclohexan, Toluol, Xylol, Tetrahydrofuran, Methyltetrahydrofuran, Dioxan, Ethylacetat, Isopropylacetat oder Dichlormethan oder Mischungen dieser Lösungsmittel verwendet werden. Das Lösungsmittel wird vorzugsweise in einer Menge von 1.0 bis 2.0 Umol eingesetzter Verbindung der allgemeinen Formel **Ia,** vorzugsweise in einer Menge von 1.4 bis 1.6 Umol eingesetzter Verbindung der allgemeinen Formel **Ia,** zugegeben.

Die Kupplung kann auch in Gegenwart einer Base erfolgen. Die Base wird vorzugsweise in einer Menge von 3.0 bis 5.0 Äquivalenten, vorzugsweise 3.8 bis 4.5 Äquivalenten, zugegeben, bezogen auf die Menge an eingesetzter Verbindung der allgemeinen Formel **Ia.** Verwendet werden können Lithiumcarbonat, Kaliumcarbonat, Natriumcarbonat, Triethylamin, Disopropylethylamin oder DBU (Diazabicyclo[5.4.0]undec-7-en), wobei Kaliumcarbonat erfindungsgemäß bevorzugt verwendet wird.

Die unter Schritt (f) beschriebene Isolierung einer Verbindung der allgemeinen Formel **Ib** kann in Form des freien Amins erfolgen, wobei **n** die Ziffer 0 darstellt.

Eine so erhaltene Verbindung kann anschließend in einem Lösungsmittel gelöst und durch Zugabe einer entsprechenden Menge Salzsäure in eine Verbindung der allgemeinen Formel **Ib** übergeführt werden, in der **n** eine der Ziffern 1, 2 oder 3, vorzugsweise die Ziffer 3, bedeutet.

Als Lösungsmittel kann dabei Wasser, Methanol, Ethanol, Propanol, Butanol, Isopropanol, Isopropylacatat, tert-Amylalkohol, Tetrahydrofuran, Methyltetrahydrofuran, Dioxan, Ethylacetat, Dichlormethan, Methylcyclohexan, Toluol oder ein Gemisch dieser Lösungsmittel verwendet werden.

Eine unter Schritt (f) erhaltene Verbindung der allgemeinen Formel **Ib,** in der **m, n, o** und **R¹** wie voranstehend erwähnt definiert sind und **R²** eine Benzyloxycarbonylgruppe darstellt, lässt sich in Gegenwart von Lithiumaluminiumhydrid in eine Verbindung der allgemeinen Formel **Ib** überführen, in der **m, n, o** und **R¹** wie voranstehend erwähnt definiert sind und **R²** eine Methylgruppe darstellt.

Bei der Kupplung in Schritt (g) werden vorzugsweise 1.0 Äquivalente einer Verbindung der allgemeinen Formel **Ib** mit 1.0 bis 1.5 Äquivalenten, vorzugsweise 1.0 bis 1.2 Äquivalenten, einer Verbindung der allgemeinen Formel **VIII** in einem Lösungsmittel und in Gegenwart einer Base umgesetzt. Als Lösungsmittel kann Wasser, Methanol, Ethanol, Propanol, Butanol, Isopropanol Aceton, Toluol, Xylol, Tetrahydrofuran, Methyltetrahydrofuran, Dioxan, Ethylacetat, Isopropylacetat oder Dichlormethan oder Mischungen dieser Lösungsmittel verwendet werden. Das Lösungsmittel wird vorzugsweise in einer Menge von 1.0 bis 2.0 Umol eingesetzter Verbindung der allgemeinen Formel **Ib,** vorzugsweise in einer Menge von 1.4 bis 1.6 Umol eingesetzter Verbindung der allgemeinen Formel **Ib,** zugegeben. Die Base wird vorzugsweise in einer Menge von 3.0 bis 5.0 Äquivalenten, vorzugsweise 3.8 bis 4.5 Äquivalenten, zugegeben, bezogen auf die Menge an eingesetzter Verbindung der allgemeinen Formel **Ib.** Verwendet werden können Lithiumcarbonat, Kaliumcarbonat, Natriumcarbonat, Natriumhydrogencarbonat, Triethylamin, Disopropylethylamin oder Diazabicyclo[5.4.0]undec-7-en (DBU), wobei Kaliumcarbonat erfindungsgemäß bevorzugt verwendet wird.

Die unter Schritt (h) beschriebene Isolierung einer Verbindung der allgemeinen Formel **I** kann in Form des freien Amins erfolgen, wobei **n** die Ziffer 0 darstellt.

Eine so erhaltene Verbindung der allgemeinen Formel **I** kann anschließend in einem Lösungsmittel gelöst und durch Zugabe einer entsprechenden Menge Salzsäure in eine Verbindung der allgemeinen Formel **I** übergeführt werden, in der **n** eine der Ziffern 1, 2 oder 3, vorzugsweise die Ziffer 3, bedeutet.

Als Lösungsmittel kann dabei Methanol, Ethanol, Propanol, Butanol, Isopropanol, Isopropylacatat, tert-Amylalkohol, Tetrahydrofuran, Methyltetrahydrofuran, Dioxan, Ethylacetat, Dichlormethan, Methylcyclohexan oder Toluolverwendet werden.

Bei den unter den Schritten (d), (f) und (h) beschriebenen Isolierungen in Form der Hydrochloride (**n** = 1, 2 oder 3), bevorzugt des Trihydrochlorids (**n** = 3), wird überwiegend eine entsprechende racemische cis-Verbindung erhalten.

Die unter Schritt (i) beschriebene Trennung der Enantiomere erfolgt in Wasser oder einem organischen Lösungsmittel oder einem Gemisch daraus. Das organische Lösungsmittel kann ausgewählt sein aus der Gruppe bestehend aus Methanol, Ethanol, Propanol, Butanol, Isopropanol, Isopropylacatat, tert-Amylalkohol, Tetrahydrofuran, Methyl-tetrahydrofuran, Dioxan, Ethylacetat, Dichlormethan, Methylcyclohexan oder Toluol, und kann in einer Menge von 1.0 bis 2.0 L/mol, bevorzugt 1.4 bis 1.6 L/mol, pro mol eingesetzter Verbindung der allgemeinen Formel **I** oder **Ia** oder **Ib** verwendet werden. Für Verbindungen, in denen **n** eine der Ziffern 1, 2 oder 3 bedeutet, wird zur Isolierung der Verbindung, in der **n** die Ziffer 0 bedeutet, eine entsprechende Menge einer Base zugegeben. Als Base können Lithiumcarbonat, Kaliumcarbonat, Natriumcarbonat oder Natriumhydrogencarbonat verwendet werden, wobei Kaliumcarbonat erfindungsgemäß bevorzugt verwendet wird.

Die chirale Säure kann in einer Menge von 0.4 bis 0.7 mol pro mol eingesetzter Verbindung der allgemeinen Formel **I, Ia** oder **Ib** verwendet werden. Dabei kann die Säure ausgewählt sein aus der Gruppe bestehend aus chiralen Aminosäuren, Weinsäure, Derivaten der Weinsäure, chiralen Sulfonsäuren wie beispielsweise (S)-(+)-Camphersulfonsäure, Camphansäure, Derivaten der Camphansäure, Mandelsäure und Äpfelsäure. Erfindungsgemäß bevorzugt wird (S)-(+)-Camphersulfonsäure verwendet.

Eine unter Schritt (k) beschriebene Umsetzung erfolgt vorzugsweise in Wasser oder einem organischen Lösungsmittel oder in einem Gemisch aus Wasser und einem organischen Lösungsmittel. Das organische Lösungsmittel kann ausgewählt sein aus der Gruppe bestehend aus Methanol, Ethanol, Propanol, Butanol, Isopropanol, Isopropylacatat, tert-Amylalkohol, Tetrahydrofuran, Methyl-tetrahydrofuran, Dioxan, Ethylacetat, Dichlormethan, Methylcyclohexan oder Toluol. Es kann in einer Menge von 4.0 bis 7.0 L/mol, bevorzugt 5.0 bis 6.5 Umol eingesetzter Verbindung der allgemeinen Formel **IX** verwendet werden.

Die Base kann ausgewählt sein aus der Gruppe bestehend aus Lithiumhydroxid, Natriumhydrioxid, Kaliumhydroxid, Lithiumcarbonat, Natriumcarbonat, Kaliumcarbonat und Kalium-*tert*.butylat. Erfindungsgemäß bevorzugt wird Natriumhydroxid, Kaliumcarbonat oder Kalium-*tert*.butylat verwendet. Sie kann in einer Menge von 1.0 bis 1.5 Äquivalenten, bevorzugt von 1.0 bis 1.1 Äquivalenten, bezogen auf die Menge an eingesetzter Verbindung der allgemeinen Formel **IX** zugegeben werden.

Eine unter Schritt (m) beschriebene Abspaltung einer Aminschutzgruppe für Verbindungen der allgemeinen **I,** in denen **m, n, o** wie voranstehend erwähnt definiert sind und mindesten einer der Reste **R¹, R²** und **R³** nicht das Wasserstoffatom bedeutet, kann nach literaturbekannten Methoden erfolgen (T.W. Greene, P.G.M. Wuts "Protective Groups in Organic Synthesis", 3rd Edition, Wiley Interscience).

Eine unter Schritt (n) beschriebene Reduktion erfolgt vorzugsweise in einem organischen Lösungsmittel. Das organische Lösungsmittel kann ausgewählt sein aus der Gruppe bestehend aus Tetrahydrofuran, Methyltetrahydrofuran, Dioxan, Methylcyclohexan, Xylol und Toluol oder einem Gemisch dieser Lösungsmittel. Es kann in einer Menge von 2.0 bis 4.0 Umol, bevorzugt 2.0 bis 3.0 Umol eingesetzter Verbindung der allgemeinen Formel I verwendet werden.

Das Reduktionsmittel kann ausgewählt werden aus der Gruppe bestehend aus komplexen Metallhydriden, Lithiumaluminiumhydrid, Diisobutylaluminiumhydrid und Natriumborhydrid, wobei Lithiumaluminiumhydrid erfindungsgemäß bevorzugt verwendet wird.

Ein zweiter Gegenstand der vorliegenden Erfindung betrifft ein voranstehend unter dem ersten Gegenstand beschriebenes Verfahren zur Herstellung von Verbindungen der allgemeinen Formel **I,** dadurch gekennzeichnet, dass
- **m**: eine der Ziffern 1 oder 2,
- **n**: eine der Ziffern 0, 1, 2 oder 3,
- **o**: eine der Ziffern 0, 1 oder 2,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- und
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

Ein dritter Gegenstand der vorliegenden Erfindung betrifft ein voranstehend unter dem ersten Gegenstand beschriebenes Verfahren zur Herstellung von Verbindungen der allgemeinen Formel **I,** dadurch gekennzeichnet, dass
- **m**: eine der Ziffern 1 oder 2,
- **n**: eine der Ziffern 0, 1 oder 2,
- **o**: eine der Ziffern 0, 1 oder 2,
- **R¹**: H, C₁₋₄-Alkyl, C₃₋₆-Cycloalkyl, C₁₋₄-Alkyl-O-C(O)-, Benzyl-O-C(O)- oder Benzyl, und
- **R²**: (
a) H,
(b) Benzyl,
(c) C₁₋₄-Alkyl-O-C(O)- oder Benzyl-O-C(O)-,und
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

Ein vierter Gegenstand der vorliegenden Erfindung betrifft ein voranstehend unter dem ersten Gegenstand beschriebenes Verfahren zur Herstellung von Verbindungen der allgemeinen Formel **I**, dadurch gekennzeichnet, dass
- **m**: eine der Ziffern 1 oder 2,
- **n**: eine der Ziffern 1 oder 2,
- **o**: eine der Ziffern 0, 1 oder 2,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,und
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

Ein fünfter Gegenstand der vorliegenden Erfindung betrifft ein voranstehend unter dem ersten Gegenstand beschriebenes Verfahren zur Herstellung von Verbindungen der allgemeinen Formel **I**, dadurch gekennzeichnet, dass
- **m**: eine der Ziffern 1 oder 2,
- **n**: die Ziffer 0,
- **o**: eine der Ziffern 0, 1 oder 2,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-, und
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

Ein sechster Gegenstand der vorliegenden Erfindung betrifft ein voranstehend unter dem ersten Gegenstand beschriebenes Verfahren zur Herstellung von Verbindungen der allgemeinen Formel I, dadurch gekennzeichnet, dass
- **m**: eine der Ziffern 1 oder 2,
- **n**: die Ziffer 3,
- **o**: eine der Ziffern 0, 1 oder 2,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-, und
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

Ein siebter Gegenstand der vorliegenden Erfindung betrifft ein voranstehend unter dem ersten Gegenstand Verfahren zur Herstellung von Verbindungen der allgemeinen Formel **l,** in der **m, n, o** und **R¹** wie voranstehend unter dem ersten, zweiten, dritten, vierten, fünften oder sechsten Gegenstand definiert sind und
- **R²**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trifluoracetyl oder Trichloracetyl und
- **R³**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trifluoracetyl oder Trichloracetyl,
deren Enantiomere und deren Diastereomere bedeuten.

Ein achter Gegenstand der vorliegenden Erfindung betrifft ein voranstehend unter dem ersten Gegenstand beschriebenes Verfahren zur Herstellung von Verbindungen der allgemeinen Formel I, dadurch gekennzeichnet, dass
- **m**: die Ziffer 1,
- **n**: eine der Ziffern 0, 1, 2 oder 3,
- **o**: eine der Ziffern 1 oder 2,
- **R¹**: H, CH₃, Benzyl, tert.Butyl-O-C(O)- oder Benzyl-O-C(O)-,
- **R²**: H, CH₃, Benzyl, tert.Butyl-O-C(O)- oder Benzyl-O-C(O)-,
- **R³**: H, CH₃, Benzyl, tert.Butyl-O-C(O)- oder Benzyl-O-C(O)-,
deren Enantiomere und deren Diastereomere bedeuten.

Ein neunter Gegenstand der vorliegenden Erfindung betrifft ein alternatives Verfahren zur Herstellung von Verbindungen der allgemeinen Formel **I** 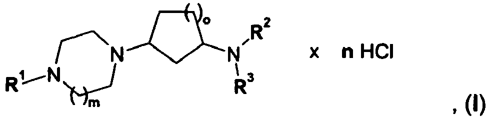 in der
- **m**: eine der Ziffern 1 oder 2,
- **n**: eine der Ziffern 0, 1, 2 oder 3,
- **o**: die Ziffer 2,
- **R¹**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
- **R²**: (a) H,
(b) C₁₋₄-Alkyl-O-C(O)-, Benzyl-O-C(O)- und
- **R³**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-
(e) Acetyl, Trifluoracetyl oder Trichloracetyl,
deren Enantiomere und deren Diastereomere bedeuten, umfassend die Schritte:
(a1) Umsetzung der Verbindung der Formel 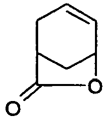 mit einer Verbindung der allgemeinen Formel III 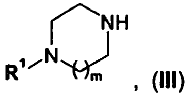 in der m und R¹ wie voranstehend erwähnt definiert sind, in Gegenwart eines Katalysators;
(a2) Reduktion einer unter Schritt (a1) erhaltenen Verbindung der allgemeinen Formel **X** 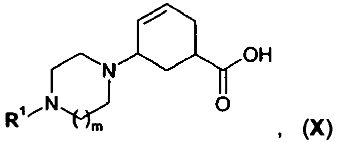 in der **m** und **R¹** wie voranstehend erwähnt definiert sind;
(a3) Umsetzung einer unter Schritt (a2) erhaltenen Verbindung der allgemeinen Formel **XI** 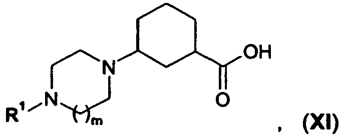 in der m und **R¹** wie voranstehend erwähnt definiert sind, in Gegenwart einer Base mit einer Azid-Quelle, beispielsweise Natriumazid oder Diphenylphosphoryl-Azid (DPPA) und Abfangen der intermediär gebildeten Verbindung der allgemeinen Formel **XII** 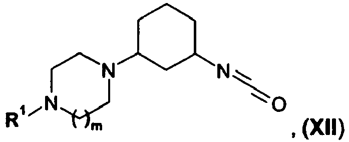 in denen **m** und **R¹** wie voranstehend erwähnt definiert sind, mit einer Verbindung der allgemeinen Formel **XIII**

   HO-R⁴, (XIII)

   in der **R⁴** ein Wasserstoffatom, eine C₁₋₄-Alkyl- oder Benzylgruppe darstellt; und
(a4) gegebenenfalls Isolierung einer unter Schritt (a3) erhaltenen Verbindung der allgemeinen Formel **Ib** 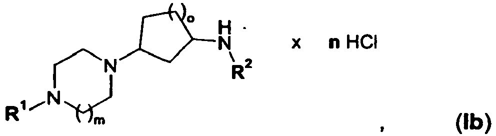 in der **m, n, o, R¹** und **R²** wie voranstehend erwähnt definiert sind.

Das Produkt jeder Stufe kann nach literaturbekannten, geeigneten Methoden, wie z.B. durch Kristallisation, Chromatographie oder Eindampfen zur Trockene, isoliert werden.

Bei der Umsetzung in Schritt (a1) werden vorzugsweise 1.0 Äquivalente 6-Oxa-bicyclo-[3.2.1]oct-3-en-7-on mit 1.0 bis 1.2 Äquivalenten einer Verbindung der allgemeinen Formel **III** in Wasser oder einem organischen Lösungsmittel oder Mischungen aus Wasser und einem organischen Lösungsmittel und in Gegenwart eines Palladiumkatalysators PdLₓ (x = 0, 1, 2, 3 oder 4), eines Platin-, Nickel-, Kupfer-, Cobalt- oder Iridumkatalysators und chiralen oder achiralen Metalliganden umgesetzt, wobei ein Palladiumkatalysator PdLₓ (x = 0, 1, 2, 3 oder 4) bevorzugt wird. Als organisches Lösungsmittel kann Methanol, Ethanol, Propanol, Butanol, Isopropylacetat, Ethylacetat, Toluol, Xylol, Tetrahydrofuran, Methyltetrahydrofuran oder Dioxan oder ein Gemisch dieser Lösungsmittel verwendet werden. Das Lösungsmittel wird vorzugsweise in einer Menge von 0.01 bis 5.0 mL/mmol, vorzugsweise in einer Menge von 0.8 bis 2.5 mL/mmol,bezogen auf die Menge an eingesetztem 6-Oxa-bicyclo[3.2.1]oct-3-en-7-on zugegeben.

Der Ligand L des Palladiumkatalysators PdLₓ **(x** = 0, 1, 2, 3 oder 4) kann ausgewählt sein aus der Gruppe bestehend aus einem Halogenid, chiralen oder achiralen Carbonsäure-, Olefin-, Phosphan-, Amin-, oder N-heterocyclischen Carbenliganden oder Kombinationen von Halogenid, chiralen oder achiralen Carbonsäure-, Olefin-, Phosphan-, Amin-, oder N-heterocyclischen Carbenliganden, wobei Phosphanliganden z.B. PPh₃, (±)-2,2'-Bis(di-phenylphosphino)-1,1'-binaphthalin, (R)-(+)-2,2'-Bis(diphenylphosphino)-1,1'-binaphthalin, (S)-(-)-2,2'-Bis(diphenylphosphino)-1,1-binaphthalin, (1R,2R)-(+)-1,2-Diaminocyclohexan-N,N'-bis(2-diphenylphosphinobenzoyl), (1S,2S)-(-)-1,2-Diaminocyclohexan-N,N'-bis(2-diphenylphosphinobenzoyl) oder Acetat, wobei Dibenzylidenaceton erfindungsgemäß bevorzugt verwendet wird.

Je nach Art/Wahl des Katalysators kann bei diesem Reaktionsschritt eine Anreicherung der racemischen cis- oder trans-Isomere der jeweils erhaltenen Verbindung der allgemeinen Formel **X** erreicht werden.

Der Palladiumkatalysator wird vorzugsweise in einer Menge von 0.001 bis 0.1 Äquivalenten, bezogen auf die Menge an eingesetztem 6-Oxa-bicyclo[3.2.1]oct-3-en-7-on, zugegeben.

Bei der Reduktion in Schritt (a2) werden vorzugsweise 1.0 Äquivalente einer Verbindung der allgemeinen Formel **X** in einem organischen Lösungsmittel in Gegenwart eines Reduktionsmittels umgesetzt. Als organisches Lösungsmittel kann Methanol, Ethanol, Propanol, Ethylacetat, Toluol, Xylol, Tetrahydrofuran oder Methyltetrahydrofuran sowie Wasser oder ein Gemisch dieser Lösungsmittel verwendet werden. Das Lösungsmittel wird vorzugsweise in einer Menge von 3 bis 6 mL/mmol eingesetzter Verbindung der allgemeinen Formel **X,** vorzugsweise von 4 bis 5 mL/mmol eingesetzter Verbindung der allgemeinen Formel **X** verwendet.

Das Reduktionsmittel kann ausgewählt sein aus der Gruppe bestehend aus Wasserstoff, Wasserstoff/Kohle/Palladium, Wasserstoff/Palladium, Wasserstoff/Raney-Nickel, Ameisensäure und Formiaten, beispielsweise Alkalimetallformiate oder Ammoniumformiat, wobei bevorzugt Wasserstoff/Kohle/Palladium verwendet wird. Es können 1 bis 5 Äquivalente, bevorzugt 1 bis 2 Äquivalente, des Reduktionsmittels zugegeben werden, jeweils bezogen auf die Menge eingesetzter Verbindung der allgemeinen Formel **X.** Vorteilhafte Bedingungen für die Hydrierung sind Temperaturen von 20 bis 60°C, vorzugsweise 25 bis 35°C, und ein Wasserstoffüberdruck von maximal 5 bar.

Nach dem Abfiltrieren des Katalysators kann das Hydrierungsprodukt durch Abdestillieren des Lösungsmittels aufkonzentriert werden.

Bei der Umsetzung in Schritt (a3) werden vorzugsweise 1.0 Äquivalente einer Verbindung der allgemeinen Formel **XI** mit 1.0 bis 1.5 Äquivalenten, vorzugsweise 1.0 bis 1.2 Äquivalenten, einer Verbindung der allgemeinen Formel **XIII**in einem Lösungsmittel und in Gegenwart einer Base umgesetzt. Als Lösungsmittel kann Toluol, Xylol, Tetrahydrofuran, Methyltetrahydrofuran, Dioxan, Ethylacetat, Isopropylacetat oder Dichlormethan oder Mischungen dieser Lösungsmittel verwendet werden. Das Lösungsmittel wird vorzugsweise in einer Menge von 2.0 bis 5.0 mL/mmol eingesetzter Verbindung der allgemeinen Formel **XI,** vorzugsweise in einer Menge von 3.0 bis 4.0 mL/mmol eingesetzter Verbindung der allgemeinen Formel **XI,** zugegeben. Die Base wird vorzugsweise in einer Menge von 1.0 bis 3.0 Äquivalenten, vorzugsweise 1.0 bis 2.0 Äquivalenten, zugegeben, bezogen auf die Menge an eingesetzter Verbindung der allgemeinen Formel **XI.** Verwendet werden können Lithiumcarbonat, Kaliumcarbonat, Natriumcarbonat, Triethylamin, Diisopropylethylamin oder DBU (Diazabicyclo[5.4.0]undec-7-en), wobei Triethylamin oder Diisopropylethylamin erfindungsgemäß bevorzugt verwendet wird.

Die unter Schritt (a4) beschriebene Isolierung einer Verbindung der allgemeinen Formel **I** kann in Form des freien Amins erfolgen, wobei n die Ziffer 0 darstellt.

Eine so erhaltene Verbindung kann anschließend in einem Lösungsmittel gelöst und durch Zugabe einer entsprechenden Menge Salzsäure in eine Verbindung der allgemeinen Formel **I** übergeführt werden, in der **n** eine der Ziffern 1, 2 oder 3 bedeutet. Als Lösungsmittel kann dabei Wasser, Methanol, Ethanol, Propanol, Isopropanol, Butanol, Ethylacetat, Isopropylacetat, Tetrahydrofuran, Methyltetrahydrofuran, Dioxan, Toluol, Acetonitril, Dichlormethan oder Methylcyclohexan verwendet werden.

Ein zehnter Gegenstand der vorliegenden Erfindung betrifft ein voranstehend unter dem neunten Gegenstand beschriebenes Verfahren zur Herstellung von Verbindungen der allgemeinen **Formel I,** dadurch gekennzeichnet, dass
- **m**: die Ziffer 1,
- **n**: eine der Ziffern 0, 1, 2 oder 3,
- **o**: die Ziffer 2,
- **R¹**: H, CH₃, Benzyl, tert.Butyl-O-C(O)- oder Benzyl-O-C(O)-,
- **R²**: H, tert.Butyl-O-C(O)- oder Benzyl-O-C(O)-,
- **R³**: H, CH₃, Benzyl, tert.Butyl-O-C(O)- oder Benzyl-O-C(O)-,
deren Enantiomere und deren Diastereomere bedeuten.

Ein elfter Gegenstand der vorliegenden Erfindung betrifft ein altematives Verfahren zur Herstellung von Verbindungen der allgemeinen Formel **I** 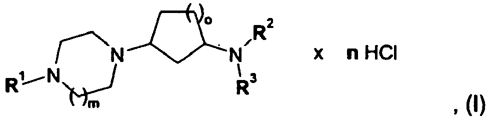 in der
- **m**: eine der Ziffern 1 oder 2,
- **n**: eine der Ziffern 0, 1, 2 oder 3,
- **o**: die Ziffer 2,
- **R¹**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
- **R²**: (a) H,
(b) C₁₋₄-Alkyl-O-C(O)-, Benzyl-O-C(O)- und
- **R³**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-
(e) Acetyl, Trifluoracetyl oder Trichloracetyl,
deren Enantiomere und deren Diastereomere bedeuten, umfassend die Schritte:
(b1) Umsetzung der Verbindung der Formel 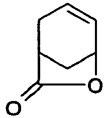 mit einer Verbindung der allgemeinen Formel **III** 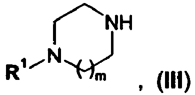 in der m und **R¹** wie voranstehend erwähnt definiert sind, in Gegenwart eines Katalysators;
(b2) Umsetzung einer unter Schritt (b1) erhaltenen Verbindung der allgemeinen Formel **X** 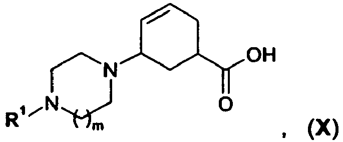 in der **m** und **R¹** wie voranstehend erwähnt definiert sind, in Gegenwart einer Base mit einer Azid-Quelle, beispielsweise Natriumazid oder Diphenylphosphoryl-Azid (DPPA), und Abfangen der intermediär gebildeten Verbindung der allgemeinen Formel **XIV** 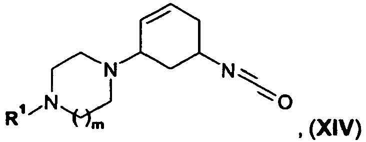 in der **m** und **R¹** wie voranstehend erwähnt definiert sind, mit einer Verbindung der allgemeinen Formel **XIII**

   HO-R⁴, (XIII)

   in der **R⁴** ein Wasserstoffatom, eine C₁₋₄-Alkyl- oder Benzylgruppe darstellt;
(b3) gegebenenfalls Isolierung einer unter Schritt (b2) erhaltenen Verbindung der allgemeinen Formel **XV** 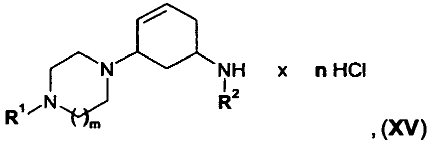 in der m, n, **R¹** und **R²** wie voranstehend erwähnt definiert sind;
(b4) Reduktion einer unter Schritt (b2) oder (b3) erhaltenen Verbindung der allgemeinen Formel **XV** 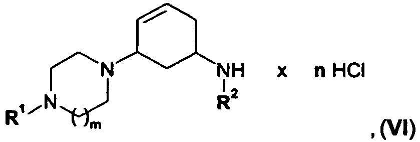 in der m und **R¹** wie voranstehend erwähnt definiert sind; und
(b5) gegebenenfalls Isolierung einer unter Schritt (b4) erhaltenen Verbindung der allgemeinen **Formel I** 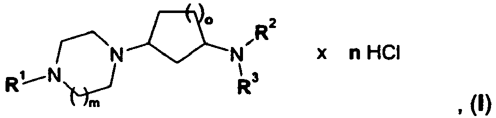 in der **m, n, R¹** und **R²** wie voranstehend erwähnt definiert sind.

Das Produkt jeder Stufe kann nach literaturbekannten, geeigneten Methoden, wie z.B. durch Kristallisation, Chromatographie oder Eindampfen zur Trockene, isoliert werden.

Bei der Umsetzung in Schritt (b1) werden vorzugsweise 1.0 Äquivalente 6-Oxa-bicyclo-[3.2.1]oct-3-en-7-on mit 1.0 bis 1.2 Äquivalenten einer Verbindung der allgemeinen Formel **III** in Wasser oder einem organischen Lösungsmittel oder Mischungen aus Wasser und einem organischen Lösungsmittel und in Gegenwart eines Palladiumkatalysators PdLₓ (x = 0, 1, 2, 3 oder 4), eines Platin-, Nickel-, Kupfer-, Cobalt- oder Iridumkatalysators und chiralen oder achiralen Metalliganden umgesetzt, wobei ein Palladiumkatalysator PdLₓ (x = 0, 1, 2, 3 oder 4) bevorzugt wird. Als organisches Lösungsmittel kann Methanol, Ethanol, Propanol, Butanol, Isopropylacetat, Ethylacetat, Toluol, Xylol, Tetrahydrofuran, Methyltetrahydrofuran oder Dioxan oder ein Gemisch dieser Lösungsmittel verwendet werden. Das Lösungsmittel wird vorzugsweise in einer Menge von 0.01 bis 5.0 mL/mmol, vorzugsweise in einer Menge von 0.8 bis 2.5 mL/mmol,bezogen auf die Menge an eingesetztem 6-Oxa-bicyclo[3.2.1]oct-3-en-7-on zugegeben.

Der Ligand L des Palladiumkatalysators PdLₓ (x = 0, 1, 2, 3 oder 4) kann ausgewählt sein aus der Gruppe bestehend aus einem Halogenid, chiralen oder achiralen Carbonsäure-, Olefin-, Phosphan-, Amin-, oder N-heterocyclischen Carbenliganden oder Kombinationen von Halogenid, chiralen oder achiralen Carbonsäure-, Olefin-, Phosphan-, Amin-, oder N-heterocyclischen Carbenliganden, wobei Phosphanliganden wie beispielsweise PPh₃, (±)-2,2'-Bis(diphenylphosphino)-1,1'-binaphthalin, (R)-(+)-2,2'-Bis(diphenylphosphino)-1,1'-binaphthalin, (S)-(-)-2,2'-Bis(diphenylphosphino)-1,1'-binaphthalin, (1R,2R)-(+)-1,2-Diaminocyclohexane-N,N'-bis(2-diphenylphosphinobenzoyl), (1S,2S)-(-)-1,2-Diaminocyclohexan-N,N'-bis(2-diphenylphosphinobenzoyl) oder Acetat, wobei Dibenzylidenaceton erfindungsgemäß bevorzugt verwendet wird.

Je nach Art/Wahl des Katalysators kann bei diesem Reaktionsschritt eine Anreicherung der racemischen cis- oder trans-Isomere der jeweils erhaltenen Verbindung der allgemeinen Formel **X** erreicht werden.

Der Palladiumkatalysator wird vorzugsweise in einer Menge von 0.001 bis 0.1 Äquivalenten, bezogen auf die Menge an eingesetztem 6-Oxa-bicyclo[3.2.1]oct-3-en-7-on, zugegeben.

Bei der Umsetzung in Schritt (b2) werden vorzugsweise 1.0 Äquivalente einer Verbindung der allgemeinen Formel **X** mit 1.0 bis 1.5 Äquivalenten, vorzugsweise 1.0 bis 1.2 Äquivalenten, einer Verbindung der allgemeinen Formel **XIII** in einem Lösungsmittel und in Gegenwart einer Base umgesetzt. Als Lösungsmittel kann Toluol, Xylol, Tetrahydrofuran, Methyltetrahydrofuran, Dioxan, Ethylacetat, Isopropylacetat oder Dichlormethan oder Mischungen dieser Lösungsmittel verwendet werden. Das Lösungsmittel wird vorzugsweise in einer Menge von 2.0 bis 5.0 mL/mmol eingesetzter Verbindung der allgemeinen Formel **X,** vorzugsweise in einer Menge von 3.0 bis 4.0 mL/mmol eingesetzter Verbindung der allgemeinen Formel **X,** zugegeben. Die Base wird vorzugsweise in einer Menge von 1.0 bis 3.0 Äquivalenten, vorzugsweise 1.0 bis 2.0 Äquivalenten, zugegeben, bezogen auf die Menge an eingesetzter Verbindung der allgemeinen Formel **X.** Verwendet werden können Lithiumcarbonat, Kaliumcarbonat, Natriumcarbonat, Triethylamin, Diisopropylethylamin oder DBU (Diazabicyclo[5.4.0]undec-7-en), wobei Triethylamin oder Diisopropylethylamin erfindungsgemäß bevorzugt verwendet wird.

Bei der Reduktion in Schritt (b4) werden vorzugsweise 1.0 Äquivalente einer Verbindung der allgemeinen Formel **XV** in einem organischen Lösungsmittel in Gegenwart eines Reduktionsmittels umgesetzt. Als organisches Lösungsmittel kann Methanol, Ethanol, Propanol, Ethylacetat, Toluol, Xylol, Tetrahydrofuran oder Methyltetrahydrofuran sowie Wasser oder ein Gemisch dieser Lösungsmittel verwendet werden. Das Lösungsmittel wird vorzugsweise in einer Menge von 3 bis 6 mL/mmol eingesetzter Verbindung der allgemeinen Formel **XV,** vorzugsweise von 4 bis 5 mL/mmol eingesetzter Verbindung der allgemeinen Formel **XV** verwendet.

Das Reduktionsmittel kann ausgewählt sein aus der Gruppe bestehend aus Wasserstoff, Wasserstoff/Kohle/Palladium, Wasserstoff/Palladium, Wasserstoff/Raney-Nickel, Ameisensäure und Formiaten, beispielsweise Alkalimetallformiate oder Ammoniumformiat, wobei bevorzugt Wasserstoff/Kohle/Palladium verwendet wird. Es können 1 bis 5 Äquivalente, bevorzugt 1 bis 2 Äquivalente, des Reduktionsmittels zugegeben werden, jeweils bezogen auf die Menge eingesetzter Verbindung der allgemeinen Formel **XV.** Vorteilhafte Bedingungen für die Hydrierung sind Temperaturen von 20 bis 60°C, vorzugsweise 25 bis 35°C, und ein Wasserstoffüberdruck von maximal 5 bar.

Nach dem Abfiltrieren des Katalysators kann das Hydrierungsprodukt durch Abdestillieren des Lösungsmittels aufkonzentriert werden.

Die unter Schritt (b5) beschriebene Isolierung einer Verbindung der allgemeinen Formel **I** kann in Form des freien Amins erfolgen, wobei n die Ziffer 0 darstellt.

Eine so erhaltene Verbindung kann anschließend in einem Lösungsmittel gelöst und durch Zugabe einer entsprechenden Menge Salzsäure in eine Verbindung der allgemeinen Formel **I** übergeführt werden, in der **n** eine der Ziffern 1 oder 2 bedeutet.

Als Lösungsmittel kann dabei Wasser, Methanol, Ethanol, Propanol, Isopropanol, Butanol, Ethylacetat, Isopropylacetat, Tetrahydrofuran, Methyltetrahydrofuran, Dioxan, Toluol, Acetonitril, Dichlormethan oder Methylcyclohexan verwendet werden.

Ein zwölfter Gegenstand der vorliegenden Erfindung betrifft ein voranstehend unter dem neunten Gegenstand beschriebenes Verfahren zur Herstellung von Verbindungen der allgemeinen Formel **I,** dadurch gekennzeichnet, dass
- **m**: die Ziffer 1,
- **n**: eine der Ziffern 0, 1, 2 oder 3,
- **o**: die Ziffer 2,
- **R¹**: H, CH₃, Benzyl, tert.Butyl-O-C(O)- oder Benzyl-O-C(O)-,
- **R²**: H, tert.Butyl-O-C(O)- oder Benzyl-O-C(O)-,
- **R³**: H, CH₃, Benzyl, tert.Butyl-O-C(O)- oder Benzyl-O-C(O)-,
deren Enantiomere und deren Diastereomere bedeuten.

Ein dreizehnter Gegenstand der vorliegenden Erfindung betrifft die Verbindungen der allgemeinen Formel I, in der
- **m**: eine der Ziffern 1 oder 2,
- **n**: eine der Ziffern 0, 1, 2 oder 3,
- **o**: eine der Ziffern 0, 1, 2 oder 3,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)- oder
(e) C₁₋₄-Alkyl-C(O)-, die mit 1, 2 oder 3 Fluor- oder Chloratomen substituiert sein kann, und
- **R³**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)- oder
(e) C₁₋₄-Alkyl-C(O)-, die mit 1, 2 oder 3 Fluor- oder Chloratomen substituiert sein kann, bedeuten,
deren Enantiomere, deren Diastereomere und deren Salze und Co-Kristalle mit chiralen Säuren, vorzugsweise deren Camphersulfonate.

Die Verbindungen der allgemeinen Formel I stellen wertvolle Ausgangsstoffe zur Synthese der Verbindungen der allgemeinen Formel **II** 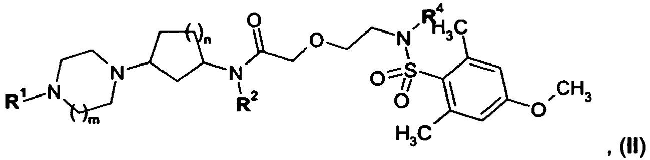 in der **m, n, R¹** und **R²** wie voranstehend erwähnt definiert sind **R⁴** ein Wasserstoffatom oder eine Methylgruppe bedeutet, dar, welche B1-antagonistische Eigenschaften besitzen.

Als weiter bevorzugte Verbindungen der allgemeinen Formel **I** seien beispielsweise folgende genannt:

| **Nr.** | **Struktur** |
|---|---|
| (1) | 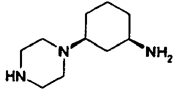 |
| (2) | 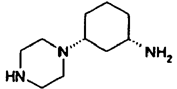 |
| (3) | 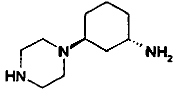 |
| (4) | 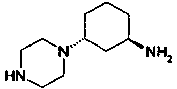 |
| (5) | 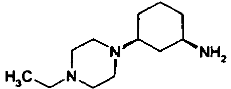 |
| (6) | 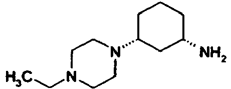 |
| (7) | 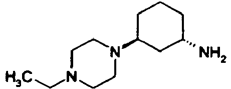 |
| (8) | 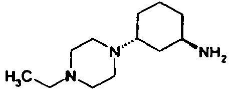 |
| (9) | 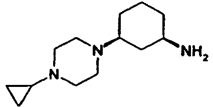 |
| (10) | 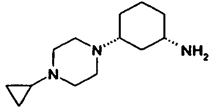 |
| (11) | 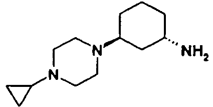 |
| (12) | 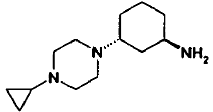 |
| (13) | 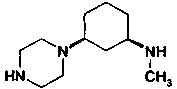 |
| (14) | 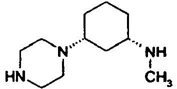 |
| (15) | 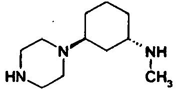 |
| (16) | 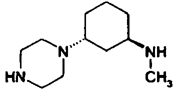 |
| (17) | 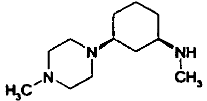 |
| (18) | 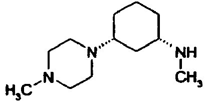 |
| (19) | 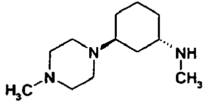 |
| (20) | 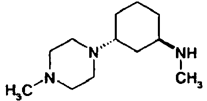 |
| (21) | 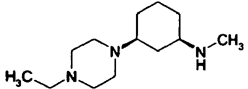 |
| (22) | 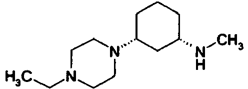 |
| (23) | 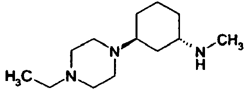 |
| (24) | 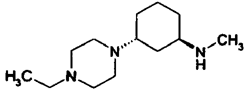 |
| (25) | 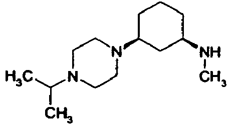 |
| (26) | 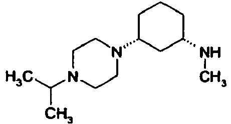 |
| (27) | 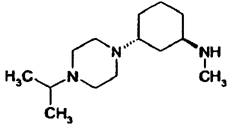 |
| (28) | 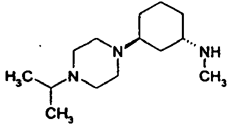 |
| (29) | 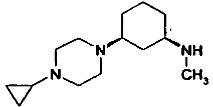 |
| (30) | 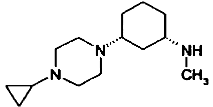 |
| (31) | 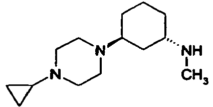 |
| (32) | 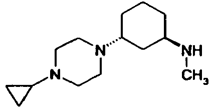 |
| (33) | 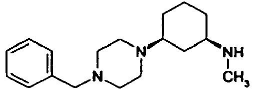 |
| (34) | 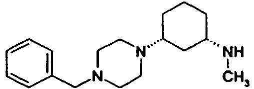 |
| (35) | 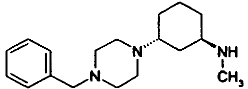 |
| (36) | 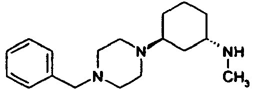 |
| (37) | 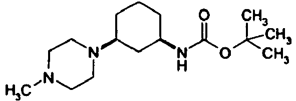 |
| (38) | 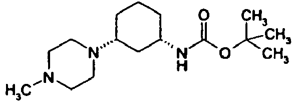 |
| (39) | 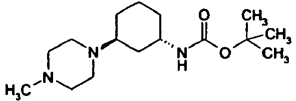 |
| (40) | 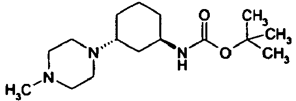 |
| (41) | 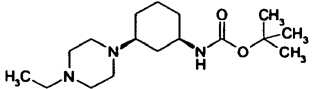 |
| (42) | 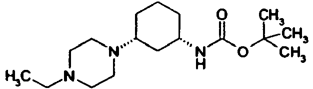 |
| (43) | 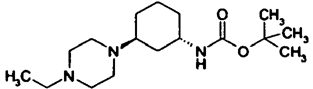 |
| (44) | 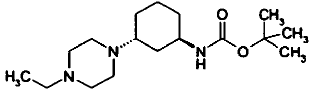 |
| (45) | 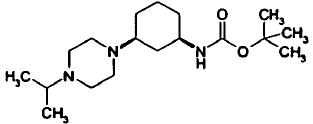 |
| (46) | 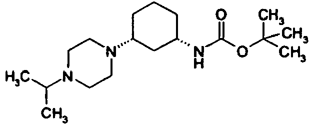 |
| (47) | 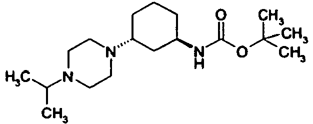 |
| (48) | 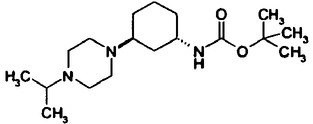 |
| (49) | 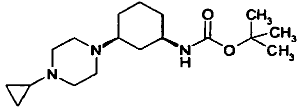 |
| (50) | 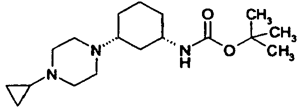 |
| (51) | 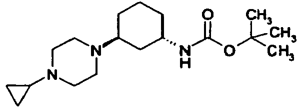 |
| (52) | 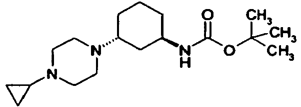 |
| (53) | 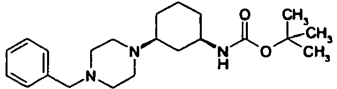 |
| (54) | 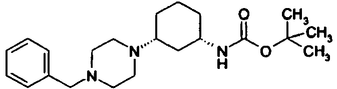 |
| (55) | 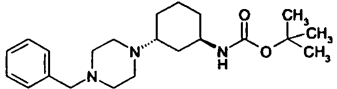 |
| (56) | 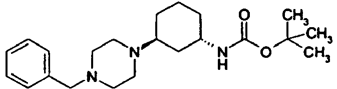 |
| (57) | 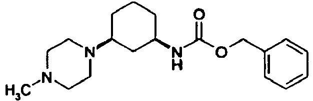 |
| (58) | 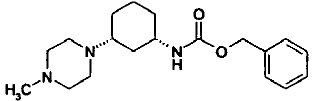 |
| (59) | 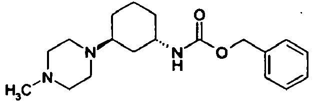 |
| (60) | 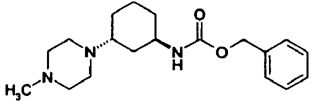 |
| (61) | 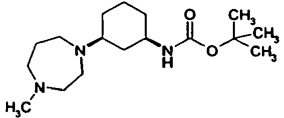 |
| (62) | 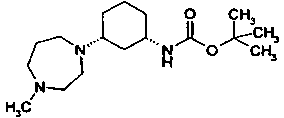 |
| (63) | 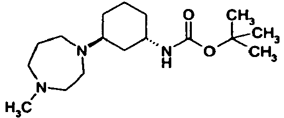 |
| (64) | 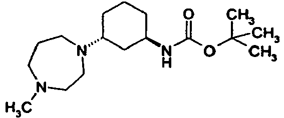 |
| (65) | 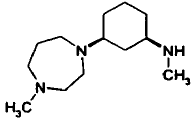 |
| (66) | 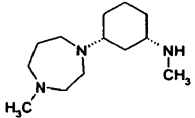 |
| (67) | 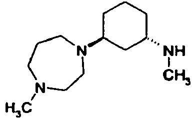 |
| (68) | 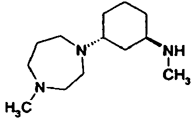 |
| (69) | 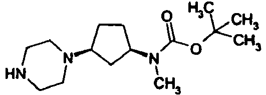 |
| (70) | 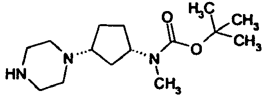 |
| (71) | 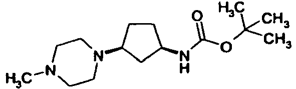 |
| (72) | 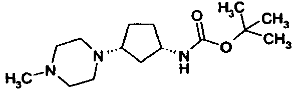 |
| (73) | 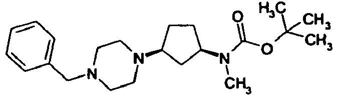 |
| (74) | 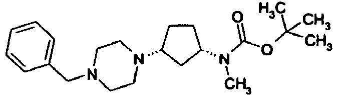 |
| (75) | 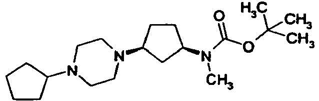 |
| (76) | 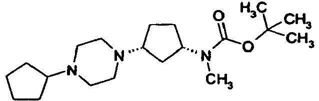 |
| (77) | 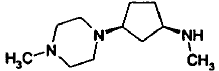 |
| (78) | 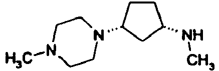 |
| (79) | 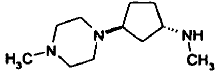 |
| (80) | 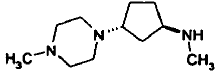 |
| (81) | 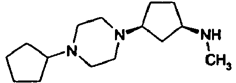 |
| (82) | 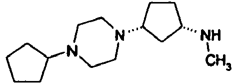 |
| (83) | 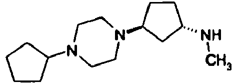 |
| (84) | 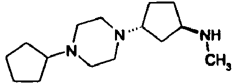 |
| (85) | 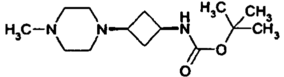 |
| (86) | 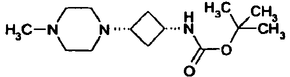 |
| (87) | 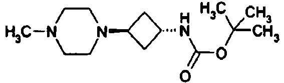 |
| (88) | 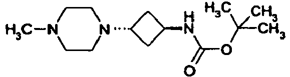 |
| (89) | 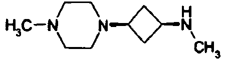 |
| (90) | 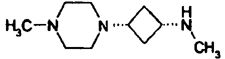 |
| (91) | 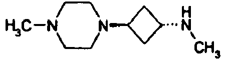 |
| (92) | 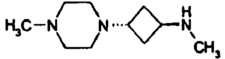 |

deren Enantiomere, deren Diastereomere und deren Salze und Co-Kristalle mit chiralen Säuren, vorzugsweise deren Camphersulfonate.

Ein vierzehnter Gegenstand der vorliegenden Erfindung betrifft die Verbindungen der allgemeinen Formel I, in der
- **m**: eine der Ziffern 1 oder 2,
- **n**: eine der Ziffern 0, 1, 2 oder 3,
- **o**: eine der Ziffern 0, 1 oder 2,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆Cycloalkyl,
(c) Benzyl,
(d) C₁₋₄-Alkyl-O-C(O)- oder Benzyl-O-C(O)- und
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
deren Enantiomere, deren Diastereomere und deren Salze und Co-Kristalle mit chiralen Säuren, vorzugsweise deren Camphersulfonate.

Ein fünfzehnter Gegenstand der vorliegenden Erfindung betrifft die Verbindungen der allgemeinen Formel **I,** in der
- **m**: eine der Ziffern 1 oder 2,
- **n**: eine der Ziffern 0, 1 oder 2,
- **o**: eine der Ziffern 0, 1 oder 2,
- **R¹**: H, C₁₋₄-Alkyl, C₃₋₆-Cycloalkyl, C₁₋₄-Alkyl-O-C(O)-, Benzyl-O-C(O)- oder Benzyl, und
- **R²**: (a) H,
(b) Benzyl,
(c) C₁₋₄-Alkyl-O-C(O)- oder Benzyl-O-C(O)-,und
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
deren Enantiomere, deren Diastereomere und deren Salze und Co-Kristalle mit chiralen Säuren, vorzugsweise deren Camphersulfonate.

Ein sechzehnter Gegenstand der vorliegenden Erfindung betrifft die Verbindungen der allgemeinen Formel **I**, in der
- **m**: eine der Ziffern 1 oder 2,
- **n**: eine der Ziffern 1 oder 2,
- **o**: eine der Ziffern 0, 1 oder 2,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
deren Enantiomere, deren Diastereomere und deren Salze und Co-Kristalle mit chiralen Säuren, vorzugsweise deren Camphersulfonate.

Ein siebzehnter Gegenstand der vorliegenden Erfindung betrifft die Verbindungen der allgemeinen Formel **I**, in der
- **m**: eine der Ziffern 1 oder 2,
- **n**: die Ziffer 0,
- **o**: eine der Ziffern 0, 1 oder 2,
- **R¹**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
deren Enantiomere, deren Diastereomere und deren Salze und Co-Kristalle mit chiralen Säuren, vorzugsweise deren Camphersulfonate.

Ein achtzehnter Gegenstand der vorliegenden Erfindung betrifft die Verbindungen der allgemeinen Formel **I**, in der
- **m**: eine der Ziffern 1 oder 2,
- **n**: die Ziffer 3,
- **o**: eine der Ziffern 0, 1 oder 2,
- **R¹**: (a) H,
(b) C₁₋₄-Alkyl, C₃₋₆Cycloalkyl,
(c) Benzyl,
(d) C₁₋₄-Alkyl-O-C(O)- oder Benzyl-O-C(O)-,
- **R²**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
- **R³**: (a) H,
(b) C₁₋₄-Alkyl, C₃₋₆-Cycloalkyl,
(c) Benzyl,
(d) C₁₋₄-Alkyl-O-C(O)- oder Benzyl-O-C(O)- bedeuten,
deren Enantiomere, deren Diastereomere und deren Salze und Co-Kristalle mit chiralen Säuren, vorzugsweise deren Camphersulfonate.

Ein neunzehnter Gegenstand der vorliegenden Erfindung betrifft die Verbindungen der allgemeinen Formel I, in der in der m, n, o und R¹ wie voranstehend unter dem dreizehnten, vierzehnten, fünfzehnten, sechzehnten, siebzehnten oder achtzehnten Gegenstand definiert sind und
- **R²**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trifluoracetyl oder Trichloracetyl und
- **R³**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trifluoracetyl oder Trichloracetyl bedeuten,
deren Enantiomere, deren Diastereomere und deren Salze und Co-Kristalle mit chiralen Säuren, vorzugsweise deren Camphersulfonate.

Ein zwanzigster Gegenstand der vorliegenden Erfindung betrifft die Verwendung der voranstehend genannten Verbindungen der allgemeinen Formel **I, in** der **m, n, o, R¹, R²** und **R³** wie voranstehend erwähnt definiert sind, als Zwischenprodukte zur Herstellung von Verbindungen der allgemeinen Formel **II,** in der m, o, **R¹** und **R²** wie voranstehend erwähnt definiert sind und **R⁴** ein Wasserstoffatom oder eine C₁₋₃-Alkylgruppe bedeutet.

Ein einundzwanzigster Gegenstand der vorliegenden Erfindung betrifft die Verbindungen der allgemeinen Formel **X** 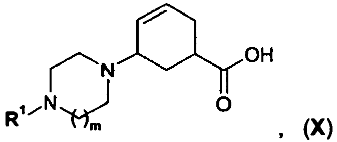 in der
- **m**: eine der Ziffern 1 oder 2 und
- **R¹**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trichloracetyl oder Trifluoracetyl bedeutet,
deren Enantiomere und deren Diastereomere sowie deren Salze und Co-Kristalle mit chiralen oder anorganischen Säuren.

Als weiter bevorzugte Verbindungen der allgemeinen Formel X seien beispielsweise folgende genannt:

| **Nr.** | **Struktur** |
|---|---|
| (1) | 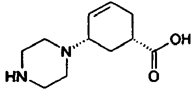 |
| (2) | 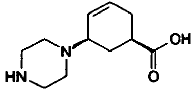 |
| (3) | 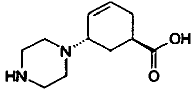 , |
| (4) | 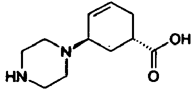 |
| (5) | 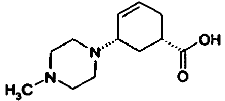 |
| (6) | 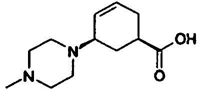 |
| (7) | 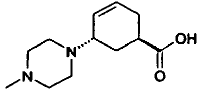 |
| (8) | 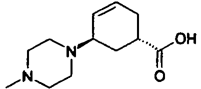 |
| (9) | 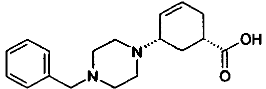 |
| (10) | 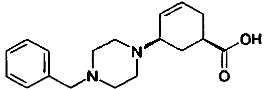 |
| (11) | 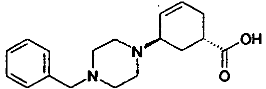 |
| (12) | 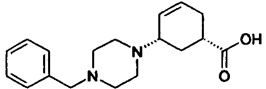 |

deren Enantiomere und deren Diastereomere sowie deren Salze und Co-Kristalle mit chiralen oder anorganischen Säuren.

Ein zweiundzwanzigster Gegenstand der vorliegenden Erfindung betrifft die Verwendung der voranstehend genannten Verbindungen der allgemeinen Formel **X,** in der m und **R¹** wie voranstehend erwähnt definiert sind, als Zwischenprodukte zur Herstellung von Verbindungen der allgemeinen Formel **II,** in der **m, o, R¹** und **R²** wie voranstehend erwähnt definiert sind und **R⁴** ein Wasserstoffatom oder eine C₁₋₃-Alkylgruppe bedeutet.

Ein dreiundzwanzigster Gegenstand der vorliegenden Erfindung betrifft die Verbindungen der allgemeinen Formel **XI** 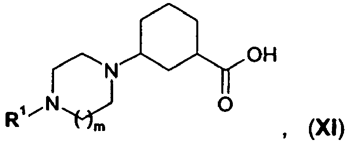 in der
- **m**: eine der Ziffern 1 oder 2 und
- **R¹**: (a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trichloracetyl oder Trifluoracetyl bedeutet,
deren Enantiomere und deren Diastereomere sowie deren Salze und Co-Kristalle mit chiralen oder anorganischen Säuren.

Als weiter bevorzugte Verbindungen der allgemeinen Formel **XI** seien beispielsweise folgende genannt:

| **Nr.** | **Struktur** |
|---|---|
| (1) | 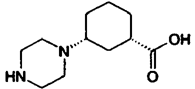 |
| (2) | 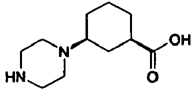 |
| (3) | 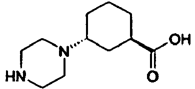 |
| (4) | 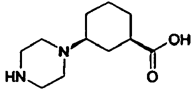 |
| (5) | 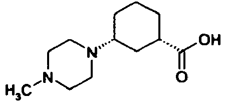 |
| (6) | 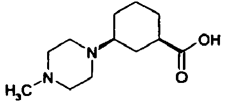 |
| (7) | 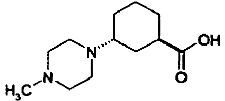 |
| (8) | 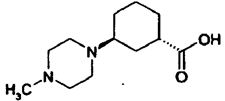 |
| (9) | 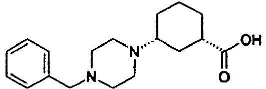 |
| (10) | 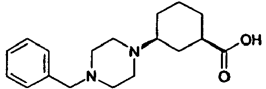 |
| (11) | 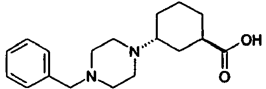 |
| (12) | 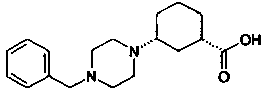 |

deren Enantiomere und deren Diastereomere sowie deren Salze und Co-Kristalle mit chiralen oder anorganischen Säuren.

Ein vierundzwanzigster Gegenstand der vorliegenden Erfindung betrifft die Verwendung der voranstehend genannten Verbindungen der allgemeinen Formel **XI,** in der **m** und **R¹** wie voranstehend erwähnt definiert sind, als Zwischenprodukte zur Herstellung von Verbindungen der allgemeinen Formel **11,** in der **m, o, R¹** und **R²** wie voranstehend erwähnt definiert sind und R⁴ ein Wasserstoffatom oder eine C₁₋₃-Alkylgruppe bedeutet.

### VERWENDETE BEGRIFFE UND DEFINITIONEN

Ebenfalls mit vom Gegenstand dieser Erfindung umfasst sind die erfindungsgemäßen Verbindungen, einschließlich deren Salze, in denen ein oder mehrere Wasserstoffatome, beispielsweise ein, zwei, drei, vier oder fünf Wasserstoffatome, durch Deuterium ausgetauscht sind.

Weiterhin mit vom Gegenstand dieser Erfindung umfasst sind die erfindungsgemäßen Verbindungen, einschließlich deren Salze, in denen ein oder mehrere Kohlenstoffatome ¹³C durch ¹⁴C ersetzt sind.

Unter dem Begriff "C₁₋₃-Alkyl" (auch soweit sie Bestandteil anderer Reste sind) werden verzweigte und unverzweigte Alkylgruppen mit 1 bis 3 Kohlenstoffatomen und unter dem Begriff "C₁₋₄-Alkyl" werden verzweigte und unverzweigte Alkylgruppen mit 1 bis 4 Kohlenstoffatomen verstanden. Beispielsweise werden hierfür genannt: Methyl, Ethyl, *n*-Propyl, *iso*-Propyl, *n*-Butyl, *iso*-Butyl oder *tert*.-Butyl. Gegebenenfalls werden für vorstehend genannten Gruppen auch die Abkürzungen Me, Et, *n*-Pr, *i*-Pr, *n*-Bu, i-Bu, *tert*-Bu etc. verwendet.

Unter dem Begriff "C₃₋₆-Cycloalkyl" (auch soweit sie Bestandteil anderer Reste sind) werden Cycloalkylgruppen mit 3 bis 6 Kohlenstoffatomen verstanden. Beispielsweise werden hierfür genannt: Cyclopropyl, Cyclobutyl, Cyclopenty oder Cyclohexyl.

Unter dem Begriff "Aminschutzgruppe" wird im Sinne der Erfindung eine Benzyl, C₁₋₄-Alkyl-O-C(O)-, Benzyl-O-C(O)-, Acetyl, Trifluoracetyl oder eine Trichloracetylgruppe verstanden.

Die Verbindungen der allgemeinen Formel I können basische Gruppen wie z.B. Aminofunktionen besitzen. Sie können daher als innere Salze, als Salze mit pharmazeutisch verwendbaren anorganischen Säuren wie beispielsweise Bromwasserstoffsäure, Phosphorsäure, Salpetersäure, Salzsäure, Schwefelsäure, Methansulfonsäure, Ethansulfonsäure, Benzolsulfonsäure, *p*-Toluolsulfonsäure oder organischen Säuren wie beispielsweise Äpfelsäure, Bernsteinsäure, Essigsäure, Fumarsäure, Maleinsäure, Mandelsäure, Milchsäure, Weinsäure oder Zitronensäure vorliegen.

Vorzugsweise können die Verbindungen der allgemeinen Formel I als Salze oder Co-Kristalle mit chiralen organischen Säuren vorliegen. Als chirale Säuren eignen sich insbesondere chirale Aminosäuren, Weinsäure, Derivate der Weinsäure, chirale Sulfonsäuren wie beispielsweise (S)-(+)-Camphersulfonsäure, Camphansäure, Derivate der Camphansäure, Mandelsäure oder Äpfelsäure, wobei (S)-(+)-Camphersulfonsäure eine herausragende Bedeutung zukommt.

Gegenstand der Erfindung sind die jeweiligen Verbindungen gegebenenfalls in Form der einzelnen optischen Isomeren, Enantiomere oder Diastereomere, Mischungen der einzelnen Enantiomeren oder Racemate, in Form der Tautomere sowie in Form der freien Basen oder der entsprechenden Säureadditionssalze.

### EXPERIMENTELLER TEIL

### Herstellung der Endverbindung

### Beispiel 1.1: 3-(4-Methyl-piperazin-1-yl)-cyclohexanon-oxim (D)

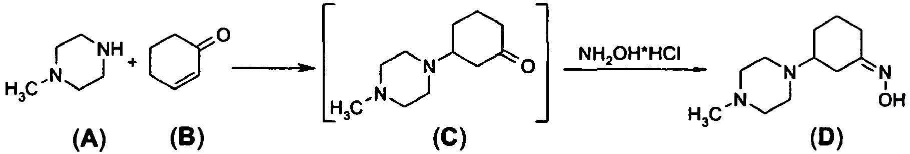

10.00 kg (104.03 mol) 2-Cyclohexenon (B) und 10.42 kg (104.03 mol) *N*-Methylpiperazin (A) wurden ca. 1 Stunde bei Raumtemperatur in 30.0 L Ethanol gerührt. Anschließend wurde das Gemisch mit 60.0 L Ethanol verdünnt und auf 0°C ± 5°C abgekühlt. Nach portionsweiser Zugabe von 16.17 kg (117.03 mol) Kaliumcarbonat und 8.13 kg (117.03 mol) Hydroxylaminhydrochlorid wurde die Reaktionsmischung für ca. 30 Minuten bei 0°C ± 5°C und anschließend ca. 30 Minuten bei Raumtemperatur weitergerührt. Die Suspension wurde filtriert und mit 40.0 L Ethanol verdünnt, bevor 110 L Lösungsmittel abdestilliert wurden. Der Rückstand wurde mit 60.0 L Tetrahydrofuran verdünnt, filtriert und mit weiteren 20.0 L Tetrahydrofuran verdünnt, bevor bei Normaldruck erneut 40.0 L Lösungsmittel abdestilliert wurden. Zum Rückstand wurden 142.0 L *n*-Heptan gegeben und das Reaktionsgemisch langsam auf Raumtemperatur abgekühlt. Bei 40°C ± 5°C wurde angeimpft. Nachdem die Suspension für ca. 12 bis 15 Stunden bei Raumtemperatur gerührt wurde, wurden weitere 102.0 L n-Heptan zugegeben, ca. 1 Stunde bei Raumtemperatur und ca. 1 Stunde bei 0°C ± 5°C gerührt. Das Produkt (D) wurde abfiltriert, zweimal mit je 36.0 L n-Heptan gewaschen und bei 50°C ± 5°C getrocknet.

| | |
|---|---|
| Ausbeute: | 18.25 kg (83% der Theorie) |
| Schmelzpunkt: | 108-110°C |

### Beispiel 1.2: rac-cis-3-(4-Methyl-piperazin-1-yl)-cyclohexylamin Trihydrochlorid (E)

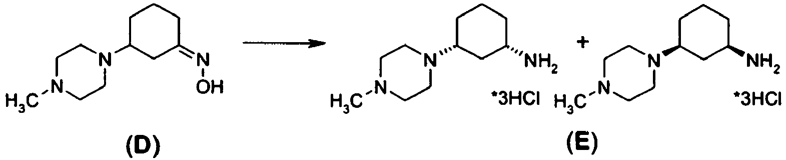

Eine Mischung aus 10.00 kg (47.32 mol) 3-(4-Methyl-piperazin-1-yl)-cyclohexanon-oxim (D), 45.0 L Toluol, 45.0 L Ethanol und 0.8 L ethanolischem Ammoniak wurde mit 1.40 kg Raney-Nickel und Wasserstoff bei ca. 5 bar und 30°C ± 5°C bis zur vollständigen Wasserstoffaufnahme hydriert. Anschließend wurde das Gemisch filtriert und mit je 20.0 L Ethanol und Methanol verdünnt, bevor das Lösungsmittel im Vakuum vollständig abdestilliert wurde. Nach Zugabe von 20.0 L Methanol wurde das Lösungsmittel im Vakuum erneut abdestilliert. Der Rückstand wurde mit 50.0 L Methanol verdünnt, auf 505°C ± 5°C erwärmt und mit 13.27 kg (141.96 mol, 10 molar) ethanolischer Salzsäure versetzt. Nach Animpfen und ca. 30 Minuten Nachrühren wurde die Suspension auf Raumtemperatur abgekühlt, das Produkt vom Lösungsmittel abgetrennt und mit 10.0 L kaltem Methanol gewaschen. Nach Umkristallisation aus 50.0 L Methanol wurde das racemische cis-Produkt (E) bei 45°C ± 5°C getrocknet.

| | |
|---|---|
| Ausbeute: | 6.24 kg (43% der Theorie) |
| Schmelzpunkt: | 254-256°C (Zersetzung) |

### Beispiel 1.3: [(1S,3R)-3-(4-Methyl-piperazin-1-yl)-cyclohexyl]-carbamidsäurebenzylester-[(1S,4R)-7,7-dimethyl-2-oxo-bicyclo[2.2.1]hept-1-yl]-methansulfonat (G)

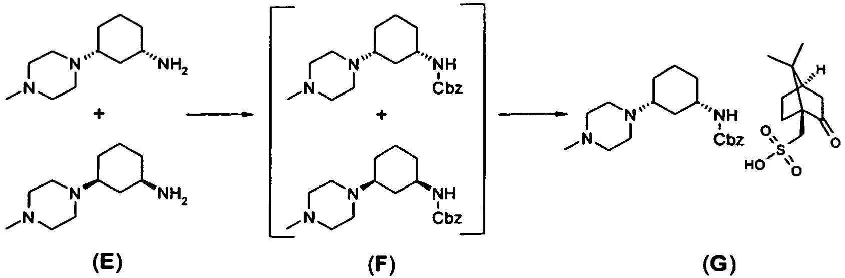

19.15 kg (138.59 mol) Kaliumcarbonat wurden in 30.0 L Wasser gelöst und mit 10.00 kg (32.61 mol) racemisches cis-3-(4-Methyl-piperazin-1-yl)-cyclohexylamin Trihydrochlorid **(E)** versetzt, bevor eine Lösung aus 8.13 kg (32.61 mol) Benzyloxycarbonyloxysuccinimid in 50.0 L Toluol bei einer Temperatur von 25°C ± 5°C zudosiert wurde. Nach ca. 30 Minuten Rühren bei 25°C ± 5°C wurden 30.0 L Wasser zugegeben und ca. 5 Minuten weitergerührt. Nach Phasentrennung wurden von der organischen Phase 40.0 L Lösungsmittel abdestilliert, bevor zum Rückstand bei 65°C ± 5°C 60.0 L Isopropylacetat zugegeben wurde. Anschließend wurde die Lösung bei Raumtemperatur zum Gemisch von 3.79 kg (16.31 mol) (1S)-(+)-Camphersulfonsäure und 0.29 L Wasser zudosiert und die Mischung solange auf Rückflusstemperatur erhitzt, bis eine Lösung vorlag. Die Reaktionslösung wurde auf 75°C ± 5°C abgekühlt, mit 10.0 g Impfkristallen angeimpft und innerhalb von ca. 3 Stunden auf Raumtemperatur abgekühlt. Nachdem die Suspension für ca. 3 Stunden bei Raumtemperatur weitergerührt wurde, wurde das Rohprodukt abgetrennt und zweimal mit je 15.0 L Isopropylacetat gewaschen. Nach Umkristallisation aus 64.0 L Isopropylacetat und 4.0 L Ethanol wurde das Produkt (G) bei 50°C ± 5°C getrocknet.

| | |
|---|---|
| Ausbeute: | 6.45 kg (34% der Theorie) |
| Schmelzpunkt: | 127-129°C |

### Beispiel 1.4: Methyl-[(1S,3R)-3-(4-methyl-piperazin-1-yl)-cyclohexyl]-amin Trihydrochlorid (I)

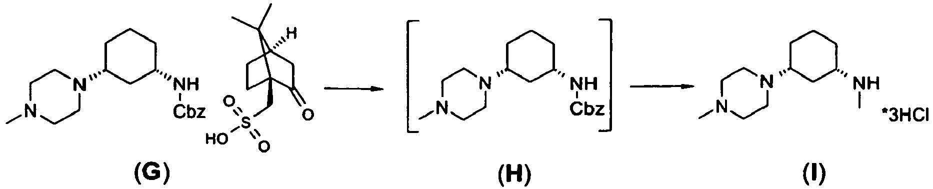

10.00 kg (17.19 mol) [(1S,3R)-3-(4-Methyl-piperazin-1-yl)-cyclohexyl]-carbamidsäure-benzylester [(1S,4R)-7,7-dimethyl-2-oxo-bicyclo[2.2.1]hept-1-yl]-methansulfonat **(G)** wurden in 50.0 L Wasser und 50.0 L Toluol suspendiert und mit 1.44 kg (18.05 mol) Natriumhydroxid-Lösung (50%ig, techn.) versetzt. Nach ca. 5 Minuten Rühren bei Raumtemperatur wurde die wässrige Phase abgetrennt und von der organischen Phase 40.0 L Lösungsmittel im Vakuum abdestilliert. Anschließend wurde der Rückstand mit 10.0 L Toluol und 16.0 L Tetrahydrofuran versetzt und die Lösung bei 85°C ± 5°C innerhalb von ca. 30 Minuten zum Gemisch aus 9.46 kg (24.93 mol) Lithiumaluminiumhydrid (10%ig in Tetrahydrofuran), 4.0 L Tetrahydrofuran und 34 L Toluol zugegeben. Nach ca. 30 Minuten Rühren wurde auf 35°C ± 5°C abgekühlt und nacheinander eine Mischung aus 0.9 L Wasser in 2.8 L Tetrahydrofuran, 0.33 kg (4.13 mol) Natriumhydroxid-Lösung (50%ig, techn.) in 0.7 L Wasser und 2.8 L Wasser zugesetzt. Anschließend wurde die Suspension filtriert und mit 16.0 L Toluol versetzt, bevor im Vakuum 80.0 L Lösungsmittel abdestilliert wurden. Zum Rückstand wurden 33.0 L Methanol zugegeben, auf 25°C ± 5°C abgekühlt und bei dieser Temperatur 4.50 kg (48.13 mol, 10M) ethanolische Salzsäure und 16.0 L Toluol zugefügt. Das Produkt (I) wurde abfiltriert und je zweimal mit einem 2:1-Gemisch aus Toluol und Methanol gewaschen und im Vakuum getrocknet.

| | |
|---|---|
| Ausbeute: | 4.58 kg (83% der Theorie) |
| Schmelzpunkt: | 279-282°C |

R_{f} = 0.81 (CH₂Cl₂/MeOH = 7/3) für **(H)**

### Beispiel 1.5: [(1S,3R)-3-(4-Methyl-piperazin-1-yl)-cyclohexyl]-carbamidsäure-tert-butylester-[(1S,4R)-7,7-dimethyl-2-oxo-bicyclo[2.2.1]hept-1-yl]-methansulfonat (K)

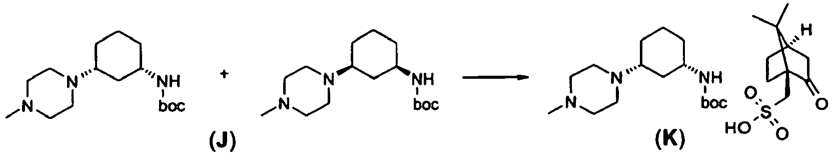

5.00 g (16.8 mmol) rac-cis-[3-(4-Methyl-piperazin-1-yl)-cyclohexyl]-carbamidsäure-*tert-*butylester (J) wurden in 40 mL Isopropylacetat suspendiert und auf 50°C erwärmt. Anschließend wurden 2 mL Ethanol und 2.00 g (8.61 mol) (1S)-(+)-Camphersulfonsäure zugegeben. Nachdem alles gelöst war, wurde die Reaktionslösung auf Raumtemperatur abgekühlt, der ausgefallene Niederschlag abfiltriert und mit Isopropylacetat gewaschen. Das farblose Produkt (K) wurde bei 50°C im Vakuum getrocknet.
Ausbeute: 2.90 g (32% der Theorie)

### Beispiel 2.1: rac-cis-5-(4-Methyl-piperazin-1-yl)-cyclohex-3-en-carbonsäure (M)

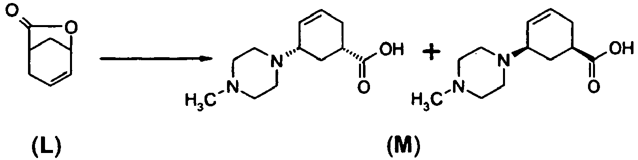

2.50 g (20.1 mmol) 6-Oxa-bicyclo[3.2.1]oct-3-en-7-on (L) wurden in 25 mL Tetrahydrofuran gelöst und auf 0°C ± 5°C gekühlt. Nach Zugabe von 223 mg (0.20 mmol) Tetrakis-(triphenylphoshin)palladium(0) und 10 mL Wasser wurden langsam 2.12 g (21.1 mmol) 1-Methylpiperazin zudosiert und das Reaktionsgemisch über Nacht auf Raumtemperatur erwärmt. Anschließend wurden 25 mL Toluol zugegeben und die wässrige Phase abgetrennt. Die organische Phase wurde mit 10 mL nachgewaschen. Die vereinigten wässrigen Phasen wurden mit Aktivkohle versetzt, kurz gerührt und filtriert. Das Filtrat wurde bei 60°C ± 5°C im Vakuum bis zur Trockene eingeengt und mit Isopropanol nachdestilliert. Der Rückstand wurde in 25 mL Ethylacetat suspendiert und 30 Minuten unter Rückfluss erhitzt. Nach Abkühlen auf Raumtemperatur wurde die Suspension abfiltriert und das Produkt **(M)** im Vakuum getrocknet.

| | |
|---|---|
| Ausbeute: | 3.67 g (82% der Theorie) |
| Schmelzpunkt: | 172-175°C |

R_{f} = 0.36 (CH₂Cl₂/MeOH/HCOOH = 7/3/0.2) für rac-cis-**(M)**
R_{f} = 0.10 (CH₂Cl₂/MeOH/HCOOH = 7/3/0.2) für rac-trans-**(M)**

### Beispiel 2.2: rac-cis-3-(4-Methyl-piperazin-1-yl)-cyclohexancarbonsäure (N)

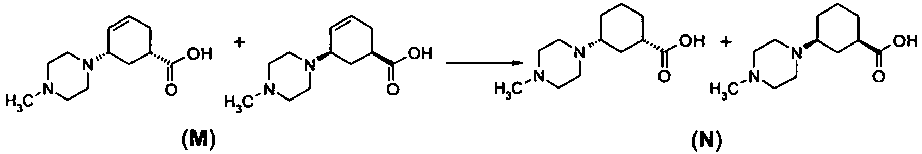

4.95 g (22.1 mmol) rac-cis-5-(4-Methyl-piperazin-1-yl)-cyclohex-3-en-carbonsäure **(M)** wurden in 100 mL Methanol gelöst und mit 0.5 g Pd/C (10%) versetzt. Anschließend wurde die Mischung bei Raumtemperatur und 50 PSI bis zur vollständigen Wasserstoffaufnahme hydriert. Das Reaktionsgemisch wurde filtriert und der Rückstand mit 20 mL Methanol gewaschen, bevor das Lösungsmittel im Vakuum vollständig entfernt wurde. Der feste Rückstand wurde mit 30 mL Ethylacetat versetzt und erneut zur Trockene eingeengt. Das erhaltene Rohprodukt **(N)** wurde in 50 mL siedendem Ethylacetat suspendiert und nach Abkühlen auf Raumtemperatur abfiltriert, mit 20 mL Ethylacetat gewaschen und im Vakuum getrocknet.

| | |
|---|---|
| Ausbeute: | 4.37 g (87% der Theorie) |
| Schmelzpunkt: | 181-184°C |

R_{f} = 0.36 (CH₂Cl₂/MeOH/HCOOH = 7/3/0.2) für **(N)**
R_{f} = 0.10 (CH₂Cl₂/MeOH/HCOOH = 7/3/0.2) für rac-trans-**(N)**

### Beispiel 2.3: rac-cis-[3-(4-Methyl-piperazin-1-yl)-cyclohexyl]-carbamidsäure-tert-butylester (O)

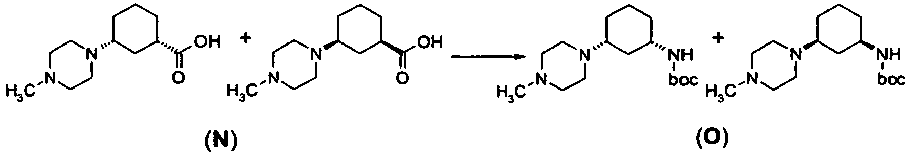

8.00 g (35.3 mmol) rac-cis-3-(4-Methyl-piperazin-1-yl)-cyclohexancarbonsäure (N) wurden in 120 mL Toluol suspendiert. Anschließend wurde das Gemisch zum Sieden erhitzt und am Wasserabscheider 13 mL Lösungsmittel entfernt. Nach Abkühlen auf 80°C ± 5°C wurden der Reihe nach 5.90 mL (42.3 mmol) Triethylamin, 10.20 g (36.3 mmol) Diphenylphosphorylazid (DPPA) und 10 mL Toluol zugegeben. Nachdem die Reaktionslösung für ca. 1 Stunde bei 80°C ± 5°C gerührt wurde, wurde sie in einen Tropftrichter überführt und langsam bei 25°C ± 5°C zur Suspension von 8.30 g (72.5 mmol) KO*t*Bu in 30 mL Toluol zudosiert. Nach ca. 1.5 Stunden Rühren bei Raumtemperatur wurden 40 mL Wasser zugegeben. Die wässrige Phase wurde abgetrennt und die organische Phase nochmals mit 40 mL Wasser gewaschen. organische Phasen wurden bis zur Trockene im Vakuum eingeengt und das Produkt **(O)** im Vakuum getrocknet.

| | |
|---|---|
| Ausbeute: | 9.14 g (87% der Theorie) |
| Schmelzpunkt: | 101-104°C |

R_{f} = 0.79 (CH₂Cl₂/MeOH/HCOOH = 7/3/0.2) für (O)

### Beispiel 2.4: rac-cis-[5-(4-Methyl-piperazin-1-yl)-cyclohex-3-enyl]-carbamidsäure-tert-butylester (Q)

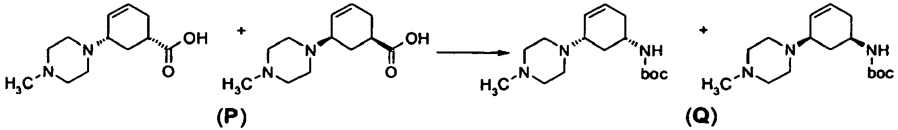

2.00 g (8.92 mmol) rac-cis-5-(4-Methyl-piperazin-1-yl)-cyclohex-3-en-carbonsäure **(P)** wurden in 50 mL Toluol suspendiert. Anschließend wurde das Gemisch zum Sieden erhitzt und am Wasserabscheider 13 mL Lösungsmittel entfernt. Nach Abkühlen auf 75°C ± 5°C wurden der Reihe nach 1.50 mL (10.8 mmol) Triethylamin und 2.75g (9.81 mmol) Diphenylphosphorylazid (DPPA) zugegeben. Nachdem die Reaktionslösung für ca. 1.5 Stunden bei 80°C ± 5°C gerührt wurde, wurde sie in einen Tropftrichter überführt und langsam unter Eiskühlung zur Suspension von 2.19 g (19.1 mmol) KO*t*Bu in 20 mL Toluol zudosiert und der Tropftrichter mit 10 mL Toluol nachgespült. Nach ca. 1.5 Std. Rühren bei Raumtemperatur wurden 40 mL Wasser zugegeben. Die wässrige Phase wurde abgetrennt und die organische Phase nochmals mit 20 mL Wasser gewaschen. Abschließend wurde die organische Phase bis zur Trockene im Vakuum eingeengt und ergab Produkt **(Q).**

| | |
|---|---|
| Ausbeute: | 2.25 g (85% der Theorie) |
| R_{f} = 0.84 (CH₂Cl₂/MeOH = 7/3) | |

### Beispiel 2.5: rac-cis-[3-(4-Methyl-piperazin-1-yl)-cyclohexyl]-carbamidsäure-benzylester (S)

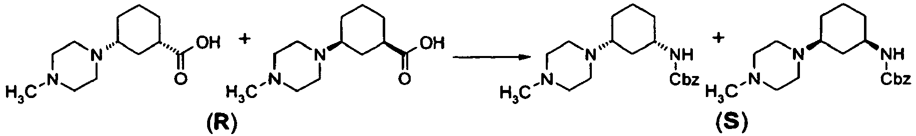

6.00 g (26.5 mmol) rac-cis-3-(4-Methyl-piperazin-1-yl)-cyclohexancarbonsäure (R) wurden in 120 mL Toluol suspendiert. Anschließend wurde das Gemisch zum Sieden erhitzt und am Wasserabscheider 13 mL Lösungsmittel entfernt. Nach Abkühlen auf 80±5°C wurden der Reihe nach 4.40 mL (42.3 mmol) Triethylamin, 10.20 g (31.5 mmol) Diphenylphosphorylazid (DPPA) und 10 mL Toluol zugegeben. Nachdem die Reaktionslösung für ca. 1 Stunde bei 80±5°C gerührt wurde, wurden 3.50 mL (32.3 mmol) Benzylalkohol und 10 mL Toluol zudosiert. Nach Abkühlen auf 40±5°C wurden 60 mL Wasser und 60 mL Ethylacatat zugegeben. Die organische Phase wurde abgetrennt und mit 60 mL Wasser gewaschen. Nach Trocknen über Na₂SO₄ wurde das Lösungsmittel im Vakuum entfernt.

| | |
|---|---|
| Ausbeute: | 7.85 g (89% der Theorie) |

## Patentansprüche

1. Verfahren zur Herstellung von Verbindungen der allgemeinen Formel I 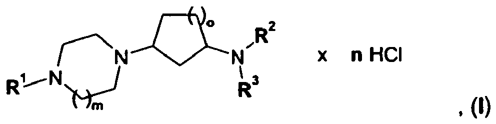 in der
**m** eine der Ziffern 1 oder 2,
**n** eine der Ziffern 0, 1, 2 oder 3,
**o** eine der Ziffern 0, 1, 2 oder 3,
**R¹**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
**R²**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)- oder
(e) C₁₋₄-Alkyl-C(O)-, die mit 1, 2 oder 3 Fluor- oder Chloratomen substituiert sein kann, und
**R³**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)- oder
(e) C₁₋₄-Alkyl-C(O)-, die mit 1, 2 oder 3 Fluor- oder Chloratomen substituiert sein kann, bedeuten,
deren Enantiomere und deren Diastereomere, umfassend die folgenden Schritte:
(a) Addition einer Verbindung der allgemeinen Formel **III** 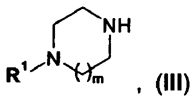 in der m und R¹ wie voranstehend erwähnt definiert sind, an eine Verbindung der allgemeinen Formel IV 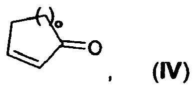 in der o wie voranstehend erwähnt definiert ist;
(b) Umsetzung einer unter Schritt (a) erhaltenen Verbindung der allgemeinen Formel V 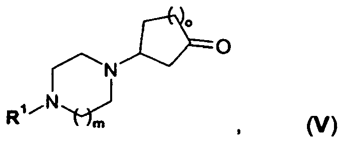 in der m, o und R¹ wie voranstehend erwähnt definiert sind, mit Hydroxylamin-Hydrochlorid;
(c) Reduktion eines unter Schritt (b) erhaltenen Oxims der allgemeinen Formel VI 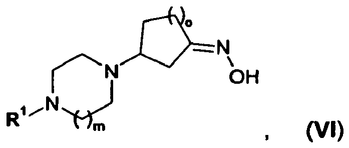 in der **m, o** und **R¹** wie voranstehend erwähnt definiert sind, in Gegenwart eines Katalysators;
(d) gegebenenfalls Isolierung einer unter Schritt (c) erhaltenen Verbindung der allgemeinen Formel **Ia** 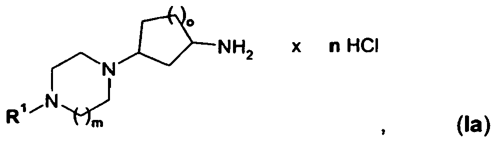 in der **m, n, o** und **R¹** wie voranstehend erwähnt definiert sind;
(e) Kupplung eines unter Schritt (c) oder (d) erhaltenen Amins der allgemeinen Formel Ia 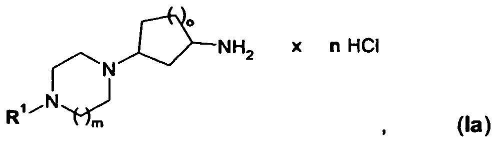 in der **m, n, o** und **R¹** wie voranstehend erwähnt definiert sind, mit einer Verbindung der allgemeinen Formel **VII**
X-R², (VII)
in der **R²** wie voranstehend erwähnt definiert ist und X eine Abgangsgruppe, beispielsweise ein Halogenatom, eine Tosylat-, Mesylat-, Triflat- oder eine Hydroxysuccinimid-gruppe, darstellt;
(f) gegebenenfalls Isolierung einer unter Schritt (e) erhaltenen Verbindung der allgemeinen Formel **Ib** 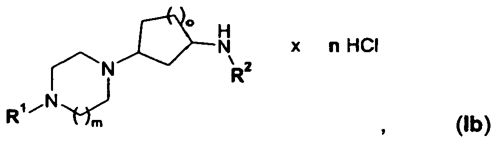 in der **m, n, o, R¹** und **R²** wie voranstehend erwähnt definiert sind;
(g) gegebenenfalls erneute Kupplung einer unter Schritt (e) oder (f) erhaltenen Verbindung der allgemeinen Formel **Ib** 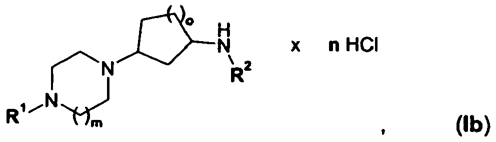 in der **m, n, o, R¹** und **R²** wie voranstehend erwähnt definiert sind, mit einer Verbindung der allgemeinen Formel VIII
X-R³, (VIII)
in der **R³** wie voranstehend erwähnt definiert ist und **X** eine Abgangsgruppe, beispielsweise ein Halogenatom, eine Tosylat-, Mesylat-, Triflat- oder eine Hydroxysuccinimid-gruppe, darstellt;
(h) gegebenenfalls Isolieren einer unter Schritt (g) erhaltenen Verbindung der allgemeinen Formel I;
(i) gegebenenfalls stereoselektive Trennung oder Anreicherung der Stereoisomere einer unter Schritt (c) oder (d) erhaltenen Verbindung der allgemeinen Formel Ia oder einer unter Schritt (e) oder (f) erhaltenen Verbindung der allgemeinen Formel Ib oder einer unter Schritt (g) oder (h) erhaltenen Verbindung der allgemeinen Formel I, durch Co-Kristallisation oder Salzbildung mit anorganischen Säuren oder chiralen Säuren;
(j) gegebenenfalls Isolierung eines oder mehrerer unter Schritt (i) erhaltenen Stereoisomere der allgemeinen Formel **IX** 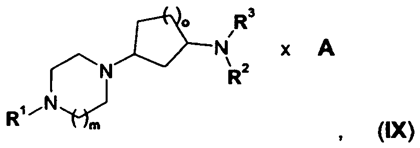 in der **m, n, o, R¹,** R² und **R³** wie voranstehend erwähnt definiert sind und A eine oder mehrere chirale Säuren oder ein oder mehrere entsprechende Anionen einer oder mehrerer anorganischer Säuren bedeutet;
(k) Umsetzung einer unter Schritt (i) oder (j) erhaltenen Verbindung der allgemeinen Formel **IX** mit einer Base;
(l) gegebenenfalls Isolieren einer stereoisomeren oder enantiomerenagreicherten Verbindung der allgemeinen Formel **I** 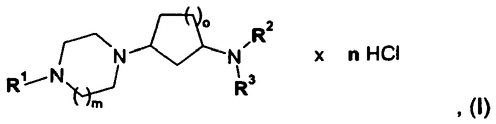 in der **m, n, o, R¹, R²** und **R³** wie voranstehend erwähnt definiert sind; und
(m) gegebenenfalls anschließendes Entfernen einer Aminschutzgruppe in einer so erhaltenen Verbindung der allgemeinen Formel I, in der **m, n** und **o** wie voranstehend erwähnt definiert sind und mindestens einer der Reste **R¹, R²** oder **R³** eine Aminschutzgruppe, beispielsweise eine Benzyl-, C₁₋₄-Alkyl-O-C(O)-, Benzyl-O-C(O)-,Acetyl-, Trifluoracetyl- oder Trichloracetylgruppe, trägt, wodurch eine Verbindung der allgemeinen Formel **I** erhalten wird, in der **m, n** und **o** wie voranstehend erwähnt definiert sind und mindestens einer der Reste **R¹, R²** oder **R³** ein Wasserstoffatom bedeutet; und
(n) gegebenenfalls Reduktion einer so erhaltenen Verbindung der allgemeinen Formel **I,** in der **m, n, o** und **R¹** wie voranstehend erwähnt definiert sind und mindestens einer der Reste **R²** oder **R³** eine C₁₋₄-Alkyl-O-C(O)- oder Benzyl-O-C(O)-Gruppe bedeutet, mit einem Reduktionsmittel, wodurch eine Verbindung der allgemeinen Formel **I** erhalten wird, in der **m, n, o** und **R¹** wie voranstehend erwähnt definiert sind und mindestens einer der Reste **R²** oder **R³** eine Methylgruppe darstellt.

2. Verfahren gemäß Anspruch 1, **dadurch gekennzeichnet, dass**
**m** eine der Ziffern 1 oder 2,
**n** eine der Ziffern 0, 1, 2 oder 3,
**o** eine der Ziffern 0, 1 oder 2,
**R¹**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
**R²**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)- und
**R³**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

3. Verfahren gemäß Anspruch 1, **dadurch gekennzeichnet, dass**
**m** eine der Ziffern 1 oder 2,
**n** eine der Ziffern 0, 1 oder 2,
**o** eine der Ziffern 0, 1 oder 2,
**R¹** H, C₁₋₄-Alkyl, C₃₋₆-Cycloalkyl, C₁₋₄-Alkyl-O-C(O)-, Benzyl-O-C(O)- oder Benzyl, und
**R²**
(a) H,
(b) Benzyl,
(c) C₁₋₄-Alkyl-O-C(O)- oder Benzyl-O-C(O)-,und
**R³**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

4. Verfahren gemäß Anspruch 1, **dadurch gekennzeichnet, dass**
**m** eine der Ziffern 1 oder 2,
**n** eine der Ziffern 1 oder 2,
**o** eine der Ziffern 0, 1 oder 2,
**R¹**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
**R²**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-, und
**R³**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

5. Verfahren gemäß Anspruch 1, **dadurch gekennzeichnet, dass**
**m** eine der Ziffern 1 oder 2,
**n** die Ziffer 0,
**o** eine der Ziffern 0, 1 oder 2,
**R¹**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
**R²**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-, und
**R³**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
deren Enantiomere und deren Diastereomere bedeuten.

6. Verfahren gemäß Anspruch 1, **dadurch gekennzeichnet, dass**
**m** eine der Ziffern 1 oder 2,
**n** die Ziffer 3,
**o** eine der Ziffern 0, 1 oder 2,
**R¹**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-,
**R²**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-, und
**R³**
(a) H,
(b) C₁₋₄-Alkyl, C₃₋₆-Cycloalkyl,
(c) Benzyl,
(d) C₁₋₄-Alkyl-O-C(O)- oder Benzyl-O-C(O)-.
deren Enantiomere und deren Diastereomere bedeuten.

7. Verfahren gemäß Anspruch 1, **dadurch gekennzeichnet, dass** l, in der m, n, o und **R¹** wie voranstehend unter Anspruch 1, 2, 3, 4, 5 oder 6 definiert sind und
**R²**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trifluoracetyl oder Trichloracetyl und
**R³**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trifluoracetyl oder Trichloracetyl,
deren Enantiomere und deren Diastereomere bedeuten.

8. Verfahren gemäß Anspruch 1, **dadurch gekennzeichnet, dass**
**m** die Ziffer 1,
**n** eine der Ziffern 0, 1, 2 oder 3,
**o** eine der Ziffern 1 oder 2,
**R¹** H, CH₃, Benzyl, tert.Butyl-O-C(O)- oder Benzyl-O-C(O)-,
**R²** H, CH₃, Benzyl, tert.Butyl-O-C(O)- oder Benzyl-O-C(O)-,
**R³** H, CH₃, Benzyl, tert.Butyl-O-C(O)- oder Benzyl-O-C(O)-,
deren Enantiomere und deren Diastereomere bedeuten.

9. Verfahren zur Herstellung von Verbindungen der allgemeinen Formel I 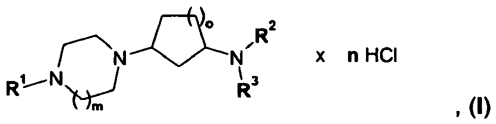 in der
**m** eine der Ziffern 1 oder 2,
**n** eine der Ziffern 0, 1, 2 oder 3,
**o** die Ziffer 2,
**R¹**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
**R²**
(a) H,
(b) C₁₋₄-Alkyl-O-C(O)-, Benzyl-O-C(O)- und
**R³**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-
(e) Acetyl, Trifluoracetyl oder Trichloracetyl,
deren Enantiomere und deren Diastereomere bedeuten, umfassend die Schritte:
(a1) Umsetzung der Verbindung der Formel 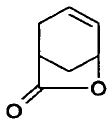 mit einer Verbindung der allgemeinen Formel III 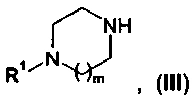 in der m und R¹ wie voranstehend erwähnt definiert sind, in Gegenwart eines Katalysators;
(a2) Reduktion einer unter Schritt (a1) erhaltenen Verbindung der allgemeinen Formel X 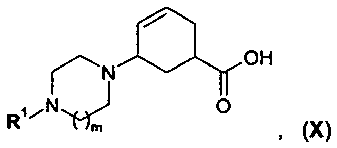 in der m und R¹ wie voranstehend erwähnt definiert sind;
(a3) Umsetzung einer unter Schritt (a2) erhaltenen Verbindung der allgemeinen Formel XI 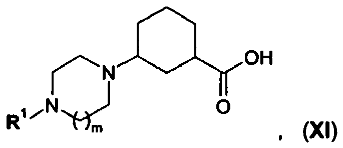 in der m und **R¹** wie voranstehend erwähnt definiert sind, in Gegenwart einer Base mit einer Azid-Quelle, beispielsweise Natriumazid oder Diphenylphosphoryl-Azid (DPPA) und Abfangen der intermediär gebildeten Verbindung der allgemeinen Formel **XII** 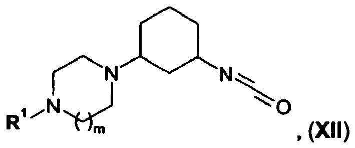 in denen **m und R¹** wie voranstehend erwähnt definiert sind, mit einer Verbindung der allgemeinen Formel **XIII**
HO-R⁴, (XIII)
in der **R⁴** ein Wasserstoffatom, eine C₁₋₄-Alkyl- oder Benzylgruppe darstellt; und
(a4) gegebenenfalls Isolierung einer unter Schritt (a3) erhaltenen Verbindung der allgemeinen Formel **Ib** 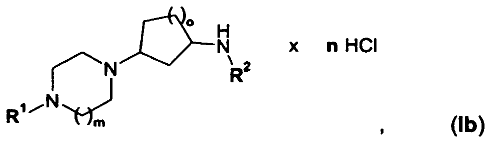 in der **m, n, o, R¹** und **R²** wie voranstehend erwähnt definiert sind.

10. Verfahren gemäß Anspruch 9, **dadurch gekennzeichnet, dass**
**m** die Ziffer 1,
**n** eine der Ziffern 0, 1, 2 oder 3,
**o** die Ziffer 2,
**R¹** H, CH₃, Benzyl, tert.Butyl-O-C(O)- oder Benzyl-O-C(O)-,
**R²** H, tert.Butyl-O-C(O)- oder Benzyl-O-C(O)-,
**R³** H, CH₃, Benzyl, tert.Butyl-O-C(O)- oder Benzyl-O-C(O)-,
deren Enantiomere und deren Diastereomere bedeuten.

11. Verfahren zur Herstellung von Verbindungen der allgemeinen Formel I 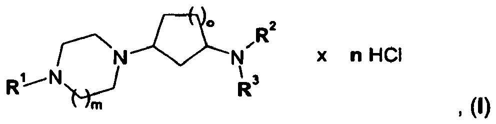 in der
**m** eine der Ziffern 1 oder 2,
**n** eine der Ziffern 0, 1, 2 oder 3,
**o** die Ziffer 2,
**R¹**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
**R²**
(a) H,
(b) C₁₋₄-Alkyl-O-C(O)-, Benzyl-O-C(O)- und
**R³**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-
(e) Acetyl, Trifluoracetyl oder Trichloracetyl,
deren Enantiomere und deren Diastereomere bedeuten, umfassend die Schritte:
(b1) Umsetzung der Verbindung der Formel 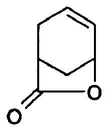 mit einer Verbindung der allgemeinen Formel III 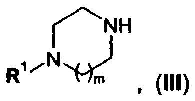 in der m und R¹ wie voranstehend erwähnt definiert sind, in Gegenwart eines Katalysators;
(b2) Umsetzung einer unter Schritt (b1) erhaltenen Verbindung der allgemeinen Formel X 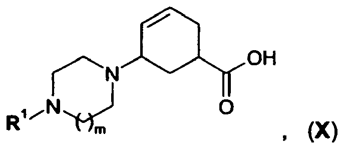 in der m und R¹ wie voranstehend erwähnt definiert sind, in Gegenwart einer Base mit einer Azid-Quelle, beispielsweise Natriumazid oder Diphenylphosphoryl-Azid (DPPA), und Abfangen der intermediär gebildeten Verbindung der allgemeinen Formel XIV 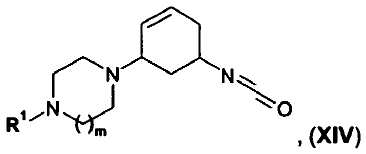 in der **m** und **R¹** wie voranstehend erwähnt definiert sind, mit einer Verbindung der allgemeinen Formel **XIII**
HO-R⁴, (XIII)
in der **R⁴** ein Wasserstoffatom, eine C₁₋₄-Alkyl- oder Benzylgruppe darstellt;
(b3) gegebenenfalls Isolierung einer unter Schritt (b2) erhaltenen Verbindung der allgemeinen Formel **XV** 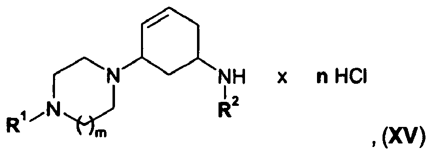 in der **m**, **n, R¹** und **R²** wie voranstehend erwähnt definiert sind;
(b4) Reduktion einer unter Schritt (b2) oder (b3) erhaltenen Verbindung der allgemeinen Formel **XV** 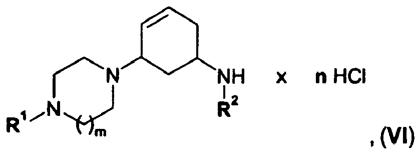 in der **m** und **R¹** wie voranstehend erwähnt definiert sind; und
(b5) gegebenenfalls Isolierung einer unter Schritt (b4) erhaltenen Verbindung der allgemeinen Formel I 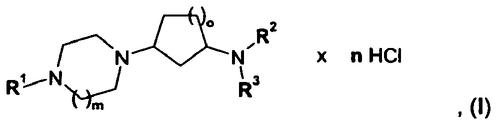 in der m, n, R¹ und R² wie voranstehend erwähnt definiert sind.

12. Verfahren gemäß Anspruch 11, **dadurch gekennzeichnet, dass**
**m** die Ziffer 1,
**n** eine der Ziffern 0, 1, 2 oder 3,
**o** die Ziffer 2,
**R¹** H, CH₃, Benzyl, tert.Butyl-O-C(O)- oder Benzyl-O-C(O)-.
**R²** H, tert.Butyl-O-C(O)- oder Benzyl-O-C(O)-,
**R³** H, CH₃, Benzyl, tert.Butyl-O-C(O)- oder Benzyl-O-C(O)-,
deren Enantiomere und deren Diastereomere bedeuten.

13. Verbindungen der allgemeinen Formel X 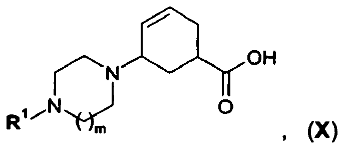 in der
**m** eine der Ziffern 1 oder 2 und
**R¹**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-.
(e) Acetyl, Trichloracetyl oder Trifluoracetyl bedeutet,
deren Enantiomere und deren Diastereomere sowie deren Salze und Co-Kristalle mit chiralen oder anorganischen Säuren.

14. Folgende Verbindungen der allgemeinen Formel X gemäß Anspruch 13:
| **Nr.** | **Struktur** |
|---|---|
| (1) | 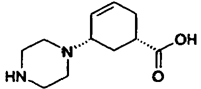 |
| (2) | 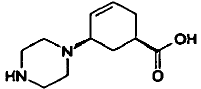 |
| (3) | 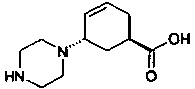 |
| (4) | 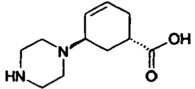 |
| (5) | 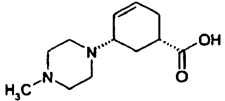 |
| (6) | 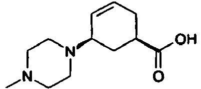 |
| (7) | 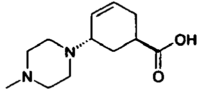 |
| (8) | 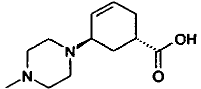 |
| (9) | 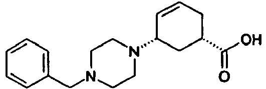 |
| (10) | 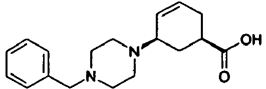 |
| (11) | 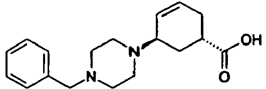 |
| (12) | 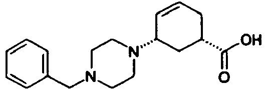 |
deren Enantiomere und deren Diastereomere sowie deren Salze und Co-Kristalle mit chiralen oder anorganischen Säuren.

15. Verbindungen der allgemeinen Formel XI 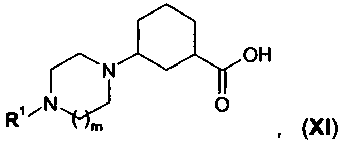 in der
**m** eine der Ziffern 1 oder 2 und
**R¹**
(a) H,
(b) C₁₋₄-Alkyl, C₃₋₆-Cycloalkyl,
(c) Benzyl,
(d) C₁₋₄-Alkyl-O-C(O)-, Benzyl-O-C(O)-,
(e) Acetyl, Trichloracetyl oder Trifluoracetyl bedeutet,
deren Enantiomere und deren Diastereomere sowie deren Salze und Co-Kristalle mit chiralen oder anorganischen Säuren.

16. Folgende Verbindungen der allgemeinen Formel **XI** gemäß Anspruch 15:
| **Nr.** | **Struktur** |
|---|---|
| (1) | 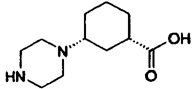 |
| (2) | 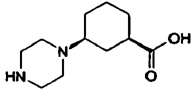 |
| (3) | 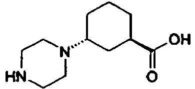 |
| (4) | 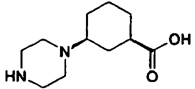 |
| (5) | 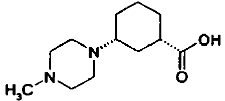 |
| (6) | 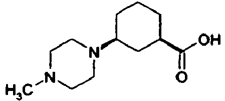 |
| (7) | 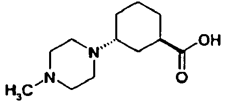 |
| (8) | 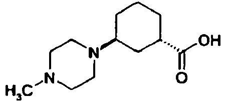 |
| (9) | 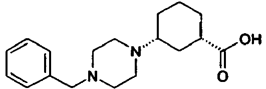 |
| (10) | 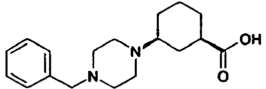 |
| (11) | 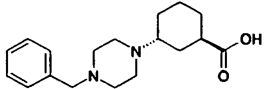 |
| (12) | 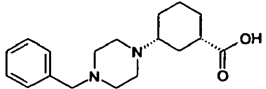 |
deren Enantiomere und deren Diastereomere sowie deren Salze und Co-Kristalle mit chiralen oder anorganischen Säuren.

## Claims

1. Method for preparing compounds of general formula I 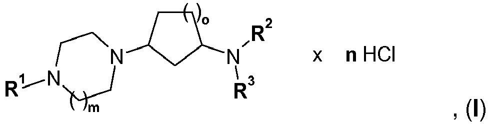 wherein
**m** denotes one of the numbers 1 or 2,
**n** denotes one of the numbers 0, 1, 2 or 3,
**o** denotes one of the numbers 0, 1, 2 or 3,
**R¹** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
**R²** denotes
(a) H,
(b) C₁₋₄-alkyl, C₃₋₆-cycloalkyl,
(c) benzyl,
(d) C₁₋₄-alkyl-O-C(O)-, benzyl-O-C(O)- or
(e) C₁₋₄-alkyl-C(O), which may be substituted by 1, 2 or 3 fluorine or chlorine atoms, and
**R³** denotes
(a) H,
(b) C₁₋₄-alkyl, C₃₋₆-cycloalkyl,
(c) benzyl,
(d) C₁₋₄-alkyl-O-C(O)-, benzyl-O-C(O)- or
(e) C₁₋₄-alkyl-C(O), which may be substituted by 1, 2 or 3 fluorine or chlorine atoms,
the enantiomers thereof and the diastereomers thereof, comprising the following steps:
(a) adding a compound of general formula III 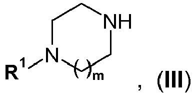 wherein m and R¹ are defined as mentioned hereinbefore, to a compound of general formula IV 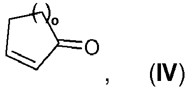 wherein o is defined as mentioned hereinbefore;
(b) reacting a compound of general formula V obtained in step (a) 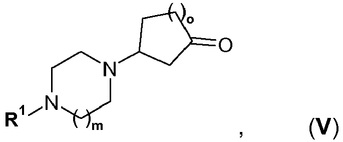 wherein m, o and R¹ are defined as mentioned hereinbefore, with hydroxylamine-hydrochloride;
(c) reducing an oxime of general formula **VI** obtained in step (b) 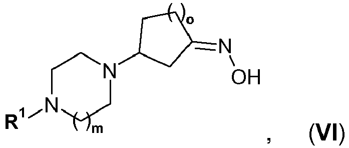 wherein **m, o** and **R¹** are defined as mentioned hereinbefore, in the presence of a catalyst;
(d) optionally isolating a compound of general formula Ia obtained in step (c) 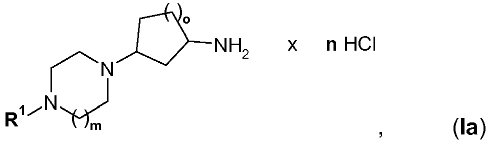 wherein **m, n, o** and **R¹** are defined as mentioned hereinbefore;
(e) coupling an amine of general formula Ia obtained in step (c) or (d) 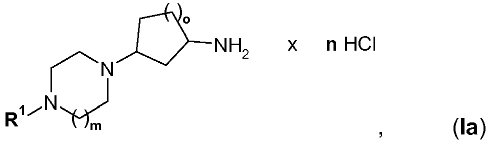 wherein **m, n, o** and **R¹** are defined as mentioned hereinbefore, to a compound of general formula VII
X-R², (VII)
wherein **R²** is defined as mentioned hereinbefore and **X** denotes a leaving group, for example a halogen atom, a tosylate, mesylate, triflate or a hydroxysuccinimide group;
(f) optionally isolating a compound of general formula **Ib** obtained in step (e) 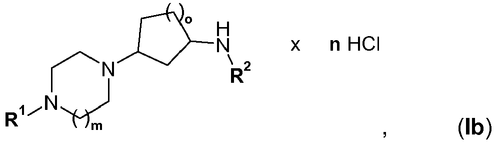 wherein **m, n, o, R¹** and **R²** are defined as mentioned hereinbefore;
(g) optionally again coupling a compound of general formula **Ib** obtained in step (e) or (f) 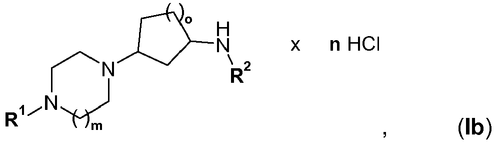 wherein **m, n, o, R¹** and **R²** are defined as mentioned hereinbefore, to a compound of general formula **VIII**
X-R³, (VIII)
wherein **R³** is defined as mentioned hereinbefore and **X** denotes a leaving group, for example a halogen atom, a tosylate, mesylate, triflate or a hydroxysuccinimide group;
(h) optionally isolating a compound of general formula I obtained in step (g);
(i) optionally stereoselectively separating or concentrating the stereoisomers of a compound of general formula **Ia** obtained in step (c) or (d) or of a compound of general formula **Ib** obtained in step (e) or (f) or of a compound of general formula I obtained in step (g) or (h), by co-crystallisation or salt formation with inorganic acids or chiral acids;
(j) optionally isolating one or more stereoisomers of general formula **IX** obtained in
step (i) 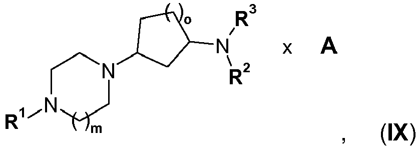 wherein **m, n, o, R¹, R²** and **R³** are defined as mentioned hereinbefore and **A** denotes one or more chiral acids or one or more corresponding anions of one or more inorganic acids;
(k) reacting a compound of general formula **IX** obtained in step (i) or (j) with a base;
(l) optionally isolating a stereoisomeric or enantiomerically enriched compound of general formula **I** 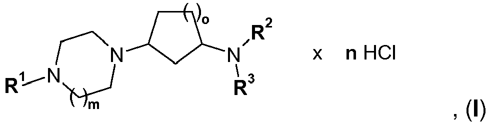 wherein **m, n, o, R¹, R²** and **R³** are defined as mentioned hereinbefore; and
(m) optionally subsequently eliminating an amine protecting group in a compound of general formula I thus obtained wherein **m, n and o** are defined as mentioned hereinbefore and at least one of the groups **R¹, R² or R³** carries an amine protecting group, for example a benzyl, C₁₋₄-alkyl-O-C(O)-, benzyl-O-C(O)-, acetyl, trifluoroacetyl or trichloroacetyl group, thus obtaining a compound of general formula **I wherein m, n and o** are defined as mentioned hereinbefore and at least one of the groups **R¹, R² or R³ denotes** a hydrogen atom; and
(n) optionally reducing a compound of general formula I thus obtained wherein **m, n, o** and **R¹** are defined as mentioned hereinbefore and at least one of the groups **R²** or **R³** denotes a C₁₋₄-alkyl-O-C(O)- or benzyl-O-C(O)- group, with a reducing agent, thus obtaining a compound of general formula I wherein **m, n, o** and **R¹** are defined as mentioned hereinbefore and at least one of the groups **R²** or **R³** denotes a methyl group.

2. Method according to claim 1, **characterised in that**
**m** denotes one of the numbers 1 or 2,
**n** denotes one of the numbers 0, 1, 2 or 3,
**o** denotes one of the numbers 0, 1 or 2,
**R¹** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
**R²** denotes
(a) H,
(b) C₁₋₄-alkyl, C₃₋₆-cycloalkyl,
(c) benzyl,
(d) C₁₋₄-alkyl-O-C(O)- or benzyl-O-C(O)- and
**R³** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
the enantiomers thereof and the diastereomers thereof.

3. Method according to claim 1, **characterised in that**
**m** denotes one of the numbers 1 or 2,
**n** denotes one of the numbers 0, 1 or 2,
**o** denotes one of the numbers 0, 1 or 2,
**R¹** denotes H, C₁₋₄-alkyl, C₃₋₆-cycloalkyl, C₁₋₄-alkyl-O-C(O)-, benzyl-O-C(O)- or benzyl, and
**R²** denotes
(a) H,
(b) benzyl,
(c) C₁₋₄-alkyl-O-C(O)- or benzyl-O-C(O)-, and
**R³** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
the enantiomers thereof and the diastereomers thereof.

4. Method according to claim 1, **characterised in that**
**m** denotes one of the numbers 1 or 2,
**n** denotes one of the numbers 1 or 2,
**o** denotes one of the numbers 0, 1 or 2,
**R¹** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
**R²** denotes
(a) H,
(b) C₁₋₄-alkyl, C₃₋₆-cycloalkyl,
(c) benzyl,
(d) C₁₋₄-alkyl-O-C(O)- or benzyl-O-C(O)-, and
**R³** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
the enantiomers thereof and the diastereomers thereof.

5. Method according to claim 1, **characterised in that**
**m** denotes one of the numbers 1 or 2,
**n** denotes the number 0,
**o** denotes one of the numbers 0, 1 or 2,
**R¹** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
**R²** denotes
(a) H,
(b) C₁₋₄-alkyl, C₃₋₆-cycloalkyl,
(c) benzyl,
(d) C₁₋₄-alkyl-O-C(O)- or benzyl-O-C(O)-, and
**R³** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
the enantiomers thereof and the diastereomers thereof.

6. Method according to claim 1, **characterised in that**
**m** denotes one of the numbers 1 or 2,
**n** denotes the number 3,
**o** denotes one of the numbers 0, 1 or 2,
**R¹** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
**R²** denotes
(a) H,
(b) C₁₋₄-alkyl, C₃₋₆-cycloalkyl,
(c) benzyl,
(d) C₁₋₄-alkyl-O-C(O)- or benzyl-O-C(O)-, and
**R³** denotes
(a) H,
(b) C₁₋₄-alkyl, C₃₋₆-cycloalkyl,
(c) benzyl,
(d) C₁₋₄-alkyl-O-C(O)- or benzyl-O-C(O)-,
the enantiomers thereof and the diastereomers thereof.

7. Method according to claim 1, **characterised in that I**, wherein **m, n, o** and **R¹** are as hereinbefore defined in claim 1, 2, 3, 4, 5 or 6 and
**R²** denotes
(a) H,
(b) C₁₋₄-alkyl, C₃₋₆-cycloalkyl,
(c) benzyl,
(d) C₁₋₄-alkyl-O-C(O)-, benzyl-O-C(O)-,
(e) acetyl, trifluoroacetyl or trichloroacetyl and
**R³** denotes
(a) H,
(b) C₁₋₄-alkyl, C₃₋₆-cycloalkyl,
(c) benzyl,
(d) C₁₋₄-alkyl-O-C(O)-, benzyl-O-C(O)-,
(e) acetyl, trifluoroacetyl or trichloroacetyl,
the enantiomers thereof and the diastereomers thereof.

8. Method according to claim 1, **characterised in that**
**m** denotes the number 1,
**n** denotes one of the numbers 0, 1, 2 or 3,
**o** denotes one of the numbers 1 or 2,
**R¹** denotes H, CH₃, benzyl, tert.butyl-O-C(O)- or benzyl-O-C(O)-,
**R²** denotes H, CH₃, benzyl, tert.butyl-O-C(O)- or benzyl-O-C(O)-,
**R³** denotes H, CH₃, benzyl, tert.butyl-O-C(O)- or benzyl-O-C(O)-,
the enantiomers thereof and the diastereomers thereof.

9. Method for preparing compounds of general formula I 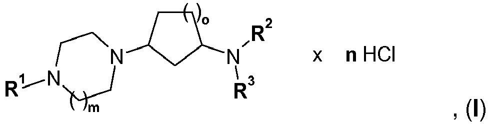 wherein
**m** denotes one of the numbers 1 or 2,
**n** denotes one of the numbers 0, 1, 2 or 3,
**o** denotes the number 2,
**R¹** denotes
(a) H,
(b) C₁₋₄-alkyl, C₃₋₆-cycloalkyl,
(c) benzyl,
(d) C₁₋₄-alkyl-O-C(O)-, benzyl-O-C(O)-,
**R²** denotes
(a) H,
(b) C₁₋₄-alkyl-O-C(O)-, benzyl-O-C(O)- and
**R³** denotes
(a) H,
(b) C₁₋₄-alkyl, C₃₋₆-cycloalkyl,
(c) benzyl,
(d) C₁₋₄-alkyl-O-C(O)-, benzyl-O-C(O)-
(e) acetyl, trifluoroacetyl or trichloroacetyl,
the enantiomers thereof and the diastereomers thereof, comprising the following steps:
(a1) reacting the compound of formula 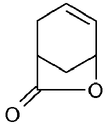 with a compound of general formula **III** 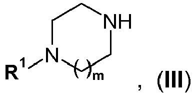 wherein m and **R¹** are defined as mentioned hereinbefore, in the presence of a catalyst;
(a2) reducing a compound of general formula X obtained in step (a1) 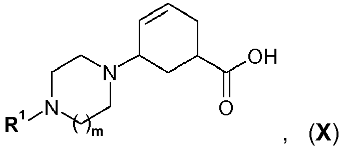 wherein m and **R¹** are defined as mentioned hereinbefore;
(a3) reacting a compound of general formula **XI** obtained in step (a2) 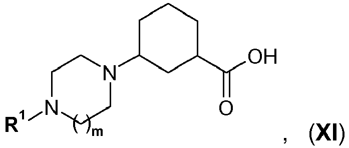 wherein m and **R¹** are defined as mentioned hereinbefore, in the presence of a base, with an azide source, for example sodium azide or diphenylphosphoryl azide (DPPA) and intercepting the resulting intermediate compound of general formula **XII** 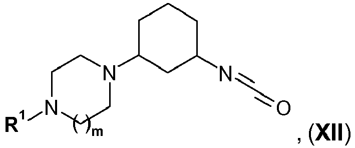 wherein m and **R¹** are defined as mentioned hereinbefore, with a compound of general formula **XIII**
HO-R⁴, (XIII)
wherein **R⁴** denotes a hydrogen atom, a C₁₋₄-alkyl or benzyl group; and
(a4) optionally isolating a compound of general formula **Ib** obtained in step (a3) 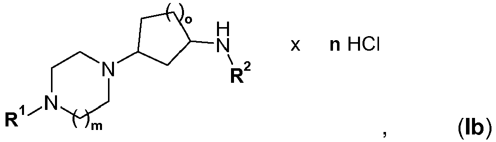 wherein **m, n, o, R¹** and **R²** are defined as mentioned hereinbefore.

10. Method according to claim 9, **characterised in that**
**m** denotes the number 1,
**n** denotes one of the numbers 0, 1, 2 or 3,
**o** denotes the number 2,
**R¹** denotes H, CH₃, benzyl, tert.butyl-O-C(O)- or benzyl-O-C(O)-,
**R²** denotes H, tert.butyl-O-C(O)- or benzyl-O-C(O)-,
**R³** denotes H, CH₃, benzyl, tert.butyl-O-C(O)- or benzyl-O-C(O)-,
the enantiomers thereof and the diastereomers thereof.

11. Method for preparing compounds of general formula I 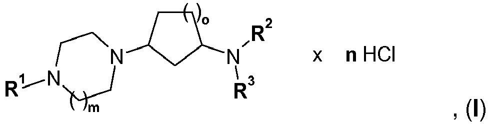 wherein
**m** denotes one of the numbers 1 or 2,
**n** denotes one of the numbers 0, 1, 2 or 3,
**o** denotes the number 2,
**R¹** denotes
(a) H,
(b) C₁₋₄-alkyl, C₃₋₆-cycloalkyl,
(c) benzyl,
(d) C₁₋₄-alkyl-O-C(O)-, benzyl-O-C(O)-,
**R²** denotes
(a) H,
(b) C₁₋₄-alkyl-O-C(O)-, benzyl-O-C(O)- and
**R³** denotes
(a) H,
(b) C₁₋₄-alkyl, C₃₋₆-cycloalkyl,
(c) benzyl,
(d) C₁₋₄-alkyl-O-C(O)-, benzyl-O-C(O)-
(e) acetyl, trifluoroacetyl or trichloroacetyl,
the enantiomers thereof and the diastereomers thereof represent, comprising the following steps:
(b1) reacting the compound of formula 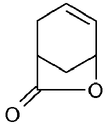 with a compound of general formula **III** 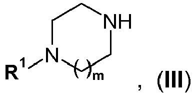 wherein m and **R¹** are defined as mentioned hereinbefore, in the presence of a catalyst;
(b2) reacting a compound of general formula X obtained in step (b1) 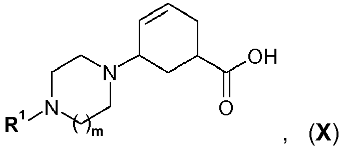 wherein m and **R¹** are defined as mentioned hereinbefore, in the presence of a base with an azide source, for example sodium azide or diphenylphosphorylazide (DPPA), and intercepting the resulting intermediate compound of general formula **XIV** 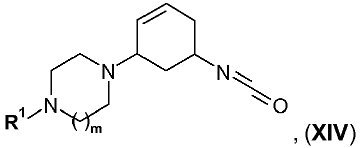 wherein m and **R¹** are defined as mentioned hereinbefore, with a compound of general formula **XIII**
HO-R⁴, (XIII)
wherein **R⁴** denotes a hydrogen atom, a C₁₋₄-alkyl or benzyl group;
(b3) optionally isolating a compound of general formula XV obtained in step (b2) 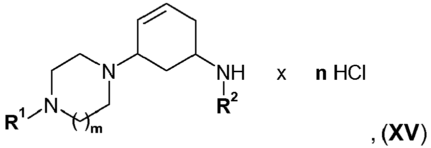 wherein **m, n, R¹** and **R²** are defined as mentioned hereinbefore;
(b4) reducing a compound of general formula XV obtained in step (b2) or (b3) 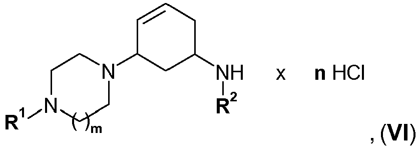 wherein **m** and **R¹** are defined as mentioned hereinbefore; and
(b5) optionally isolating a compound of general formula **I** obtained in step (b4) 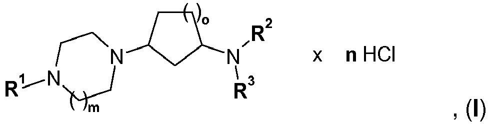 wherein **m, n, R¹** and **R²** are defined as mentioned hereinbefore.

12. Method according to claim 11, **characterised in that**
**m** denotes the number 1,
**n** denotes one of the numbers 0, 1, 2 or 3,
**o** denotes the number 2,
**R¹** denotes H, CH₃, benzyl, tert.butyl-O-C(O)- or benzyl-O-C(O)-,
**R²** denotes H, tert.butyl-O-C(O)- or benzyl-O-C(O)-,
**R³** denotes H, CH₃, benzyl, tert.butyl-O-C(O)- or benzyl-O-C(O)-,
the enantiomers thereof and the diastereomers thereof.

13. Compounds of general formula X 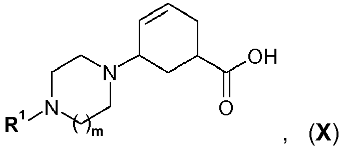 wherein
**m** denotes one of the numbers 1 or 2 and
**R¹** denotes
(a) H,
(b) C₁₋₄-alkyl, C₃₋₆-cycloalkyl,
(c) benzyl,
(d) C₁₋₄-alkyl-O-C(O)-, benzyl-O-C(O)-,
(e) acetyl, trichloroacetyl or trifluoroacetyl,
the enantiomers thereof and the diastereomers thereof as well as the salts and co-crystals thereof with chiral or inorganic acids.

14. The following compounds of general formula **X** according to claim 13:
| No. | Structure |
|---|---|
| **(1)** | 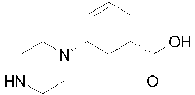 |
| **(2)** | 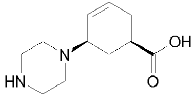 |
| **(3)** | 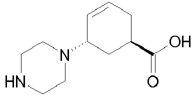 |
| **(4)** | 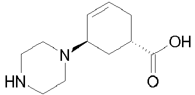 |
| **(5)** | 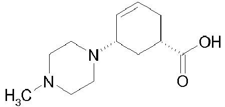 |
| **(6)** | 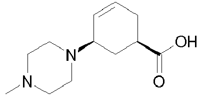 |
| **(7)** | 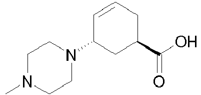 |
| **(8)** | 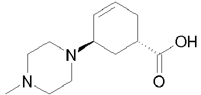 |
| **(9)** | 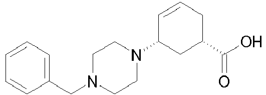 |
| **(10)** | 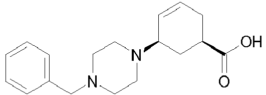 |
| **(11)** | 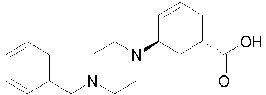 |
| **(12)** | 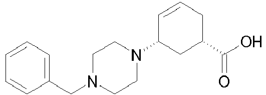 |
the enantiomers thereof and the diastereomers thereof as well as the salts and co-crystals thereof with chiral or inorganic acids.

15. Compounds of general formula **XI** 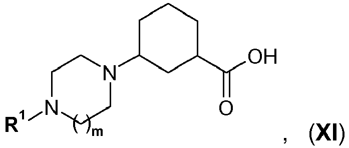 wherein
**m** denotes one of the numbers 1 or 2 and
**R¹** denotes
(a) H,
(b) C₁₋₄-alkyl, C₃₋₆-cycloalkyl,
(c) benzyl,
(d) C₁₋₄-alkyl-O-C(O)-, benzyl-O-C(O)-,
(e) acetyl, trichloroacetyl or trifluoroacetyl,
the enantiomers thereof and the diastereomers thereof as well as the salts and co-crystals thereof with chiral or inorganic acids.

16. The following compounds of general formula **XI** according to claim 15:
| **No.** | Structure |
|---|---|
| **(1)** | 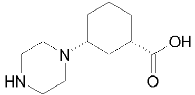 |
| **(2)** | 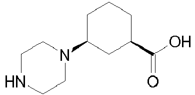 |
| **(3)** | 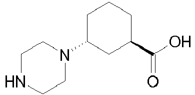 |
| **(4)** | 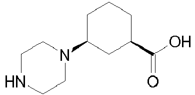 |
| **(5)** | 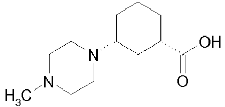 |
| **(6)** | 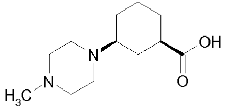 |
| **(7)** | 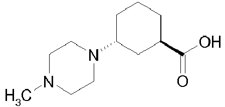 |
| **(8)** | 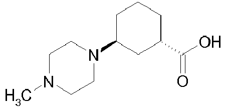 |
| **(9)** | 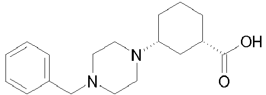 |
| **(10)** | 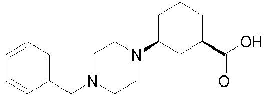 |
| **(11)** | 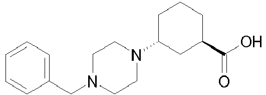 |
| **(12)** | 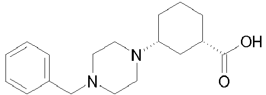 |
the enantiomers thereof and the diastereomers thereof as well as the salts and co-crystals thereof with chiral or inorganic acids.

## Revendications

1. Procédé de production de composés de formule générale I 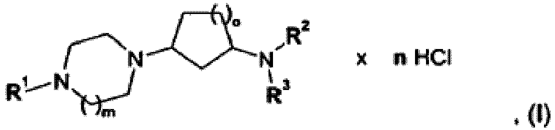 dans laquelle
m désigne l'un des chiffes 1 ou 2,
n désigne l'un des chiffres 0, 1, 2 ou 3,
o désigne l'un des chiffres 0, 1, 2 ou 3,
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-,
R² désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)- ou
(e) un groupe alkyle en C₁ à C₄-C(O)-qui peut être substitué par 1, 2 ou 3 atomes de fluor ou de chlore, et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)- ou
(e) un groupe alkyle en C₁ à C₄-C(O)- qui peut être substitué par 1, 2 ou 3 atomes de fluor ou de chlore,
leurs énantiomères et leurs diastéréomères, comprenant les étapes suivantes :
(a) addition d'un composé de formule générale III 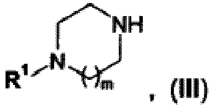 dans laquelle m et R¹ sont définis comme mentionné précédemment, à un composé de formule générale IV 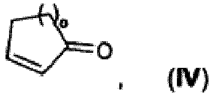 dans laquelle o est défini comme mentionné précédemment;
(b) réaction d'un composé obtenu à l'étape (a) de formule générale V 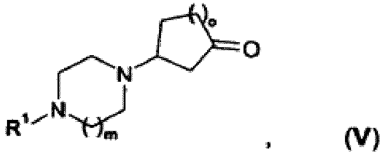 dans laquelle m, o et R¹ sont définis comme mentionné précédemment, avec du chlorhydrate d'hydroxylamine ;
(c) réduction d'une oxime obtenue à l'étape (b) de formule générale VI 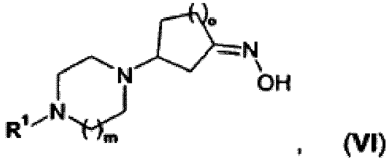 dans laquelle m, o et R¹ sont définis comme mentionné précédemment, en présence d'un catalyseur ;
(d) éventuellement, isolement d'un composé obtenu à l'étape (c) de formule générale Ia 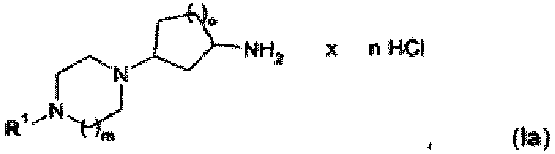 dans laquelle m, n o et R¹ sont définis comme mentionné précédemment ;
(e) couplage d'une amine obtenue à l'étape (c) ou (d) de formule générale Ia 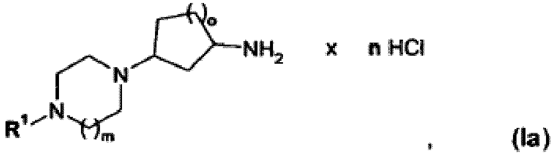 dans laquelle m, n, o et R¹ sont définis comme mentionné précédemment, avec un composé de formule générale VII
X-R², (VII)
dans laquelle R² est défini comme mentionné précédemment et X représente un groupe partant, par exemple un atome d'halogène, un groupe tosylate, mésylate, triflate ou hydroxysuccinimide ;
(f) éventuellement, isolement d'un composé obtenu à l'étape (e) de formule générale Ib 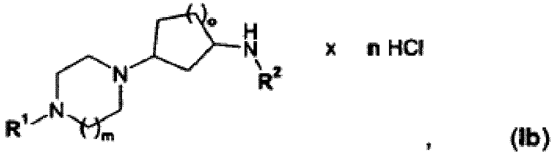 dans laquelle m, n, o, R¹ et R² sont définis comme mentionné précédemment ;
(g) éventuellement, nouveau couplage d'un composé obtenu à l'étape (e) ou (f) de formule générale Ib 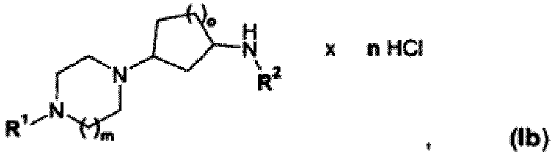 dans laquelle m, n, o, R¹ et R² sont définis comme mentionné précédemment, avec un composé de formule générale VIII
**X-R³**, (VIII)
dans laquelle R³ est défini comme mentionné précédemment et X représente un groupe partant, par exemple un atome d'halogène, un groupe tosylate, mésylate, triflate ou hydroxysuccinimide ;
(h) éventuellement, isolement d'un composé obtenu à l'étape (g) de formule générale I ;
(i) éventuellement, séparation stéréosélective ou enrichissement des stéréo-isomères d'un composé obtenu à l'étape (c) ou (d) de formule générale Ia ou d'un composé obtenu à l'étape (e) ou (f) de formule générale Ib, ou d'un composé obtenu à l'étape (g) ou (h) de formule générale I, par co-cristallisation ou formation de sel avec des acides anorganiques ou des acides chiraux ;
(j) éventuellement, isolement d'un ou plusieurs stéréo-isomères obtenus à l'étape (i) de formule générale IX 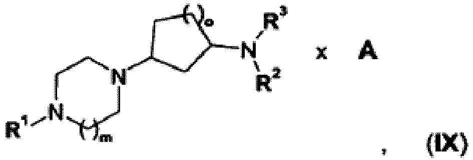 dans laquelle m, n, o, R¹, R² et R³ sont définis comme mentionné précédemment et A désigne un ou plusieurs acides chiraux ou un ou plusieurs anions correspondants d'un ou plusieurs acides anorganiques ;
(k) réaction d'un composé obtenu à l'étape (i) ou (j) de formule générale IX avec une base ;
(l) éventuellement, isolement d'un composé stéréo-isomère ou riche en énantiomère de formule générale I 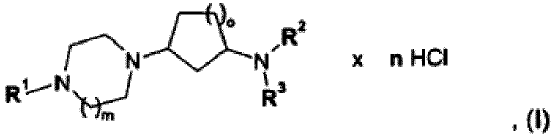 dans laquelle m, n, o, R¹, R² et R³ sont définis comme mentionné précédemment ; et
(m) éventuellement, élimination consécutive d'un groupe protecteur amine dans un composé ainsi obtenu de formule générale I, dans laquelle m, n et o sont définis comme mentionné précédemment et au moins l'un des radicaux R¹, R² ou R³ porte un groupe protecteur amine, par exemple un groupe benzyle, alkyle en C₁ à C₄-O-C(O)-, benzyle-OC(O)- acétyle, trifluoroacétyle ou trichloroacétyle, un composé de formule générale I étant ainsi obtenu, dans lequel m, n et o sont définis comme mentionné précédemment et au moins l'un des radicaux R¹, R² ou R³ désigne un atome d'hydrogène ; et
(n) éventuellement, réduction d'un composé ainsi obtenu de formule générale I, dans laquelle m, n, o et R¹ sont définis comme mentionné précédemment et au moins l'un des radicaux R² ou R³ désigne un groupe alkyle en C₁ ou C₄-O-C(O)- ou benzyle-OC(0)-, avec un agent de réduction, un composé de formule générale I étant ainsi obtenu, dans laquelle m, n, o et R¹ sont définis comme mentionné précédemment et au moins l'un des radicaux R² ou R³ représente un groupe méthyle.

2. Procédé selon la revendication 1, **caractérisé en ce que**
m désigne l'un des chiffes 1 ou 2,
n désigne l'un des chiffres 0, 1, 2 ou 3,
o désigne l'un des chiffres 0, 1 ou 2,
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-,
R² désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)- et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-
leurs énantiomères et leurs diastéréomères.

3. Procédé selon la revendication 1, **caractérisé en ce que**
m désigne l'un des chiffes 1 ou 2,
n désigne l'un des chiffres 0, 1 ou 2,
o désigne l'un des chiffres 0, 1 ou 2,
R¹ désigne H, un groupe alkyle en C₁ à C₄, cycloalkyle en C₃ à C₆, alkyle en C₁ à C₄-O-C(O)-, benzyle-O-C(O)- ou benzyle, et
R² désigne
(a) H,
(b) un groupe benzyle,
(c) un groupe alkyle en C₁ à C₄-O-C(O)- ou benzyle-O-C(O)- et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-
leurs énantiomères et leurs diastéréomères.

4. Procédé selon la revendication 1, **caractérisé en ce que**
m désigne l'un des chiffes 1 ou 2,
n désigne l'un des chiffres 1 ou 2,
o désigne l'un des chiffres 0, 1 ou 2,
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-,
R² désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)- et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-
leurs énantiomères et leurs diastéréomères.

5. Procédé selon la revendication 1, **caractérisé en ce que**
m désigne l'un des chiffes 1 ou 2,
n désigne le chiffre 0,
o désigne l'un des chiffres 0, 1 ou 2,
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-,
R² désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)- et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-
leurs énantiomères et leurs diastéréomères.

6. Procédé selon la revendication 1, **caractérisé en ce que**
m désigne l'un des chiffes 1 ou 2,
n désigne le chiffre 3,
o désigne l'un des chiffres 0, 1 ou 2,
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-,
R² désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)- et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-
leurs énantiomères et leurs diastéréomères.

7. Procédé selon la revendication 1, **caractérisé par** la formule I, dans laquelle m, n, o et
R¹ sont définis précédemment à la revendication 1, 2, 3, 4, 5 ou 6 et
R² désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)-
(e) un groupe acétyle, trifluoroacétyle ou trichloroacétyle et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)- ou benzyle-O-C(O)-
(e) un groupe acétyle, trifluoroacétyle ou trichloroacétyle
leurs énantiomères et leurs diastéréomères.

8. Procédé selon la revendication 1, **caractérisé en ce que**
m désigne le chiffre 1,
n désigne l'un des chiffres 0, 1, 2 ou 3,
o désigne l'un des chiffres 1 ou 2,
R¹ désigne H, CH₃, un groupe benzyle, tert. butyl-O-C(O)- ou benzyle-O-C(O)-,
R² désigne H, CH₃, un groupe benzyle, tert. butyl-O-C(O)- ou benzyle-O-C(O)-,
R³ désigne H, CH₃, un groupe benzyle, tert. butyl-O-C(O)- ou benzyle-O-C(O)-,
leurs énantiomères et leurs diastéréomères.

9. Procédé de production de composés de formule générale I 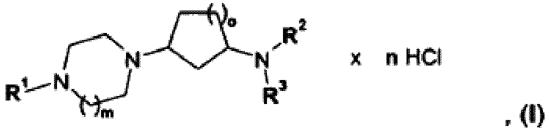 dans laquelle
m désigne l'un des chiffres 1 ou 2,
n désigne l'un des chiffres 0, 1, 2 ou 3,
o désigne le chiffre 2,
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)-,
R² désigne
(a) H,
(b) un groupe alkyle en C₁ à C₄-O-C(O)-, benzyle-O-C(O)- et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)-
(e) acétyle, trifluoroacétyle ou trichloroacétyle,
leurs énantiomères et leurs diastéréomères, comprenant les étapes de :
(a1) réaction du composé de formule 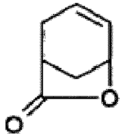 avec un composé de formule générale III 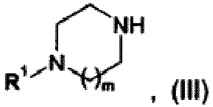 dans laquelle m et R¹ sont définis comme mentionné précédemment, en présence d'un catalyseur ;
(a2) réduction d'un composé obtenu à l'étape (a1) de formule générale X 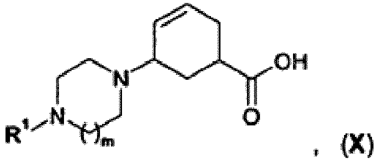 dans laquelle m et R¹ sont définis comme mentionné précédemment ;
(a3) réaction d'un composé obtenu à l'étape (a2) de formule générale XI 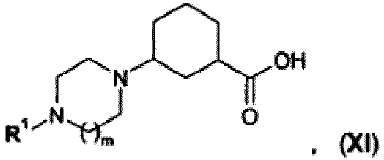 dans laquelle m et R¹ sont définis comme mentionné précédemment, en présence d'une base avec une source d'azoture, par exemple l'azoture de sodium ou l'azoture de diphénylphosphoryle (DPPA) et capture du composé formé de façon intermédiaire de formule générale XII 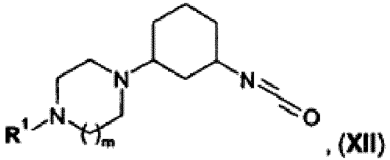 dans laquelle m et R¹ sont définis comme mentionné précédemment, avec un composé de formule générale
**HO-R⁴,** (XIII)
dans laquelle R⁴ représente un atome d'hydrogène, un groupe alkyle en C₁ à C₄ ou benzyle ; et
(a4) éventuellement, isolement d'un composé obtenu à l'étape (a3) de formule générale Ib 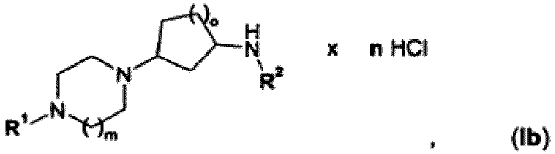 dans laquelle m, n, o, R¹ et R² sont définis comme mentionné précédemment.

10. Procédé selon la revendication 9, **caractérisé en ce que**
m désigne le chiffre 1,
n désigne l'un des chiffres 0, 1, 2 ou 3,
o désigne le chiffre 2,
R¹ désigne H, CH₃, un groupe benzyle, tert. butyl-O-C(O)- ou benzyle-O-C(O)-,
R² désigne H, un groupe tert. butyl-O-C(O)- ou benzyle-O-C(O)-,
R³ désigne H, CH₃, un groupe benzyle, tert. butyl-O-C(O)- ou benzyle-O-C(O)-,
leurs énantiomères et leurs diastéréomères.

11. Procédé de production de composés de formule générale I 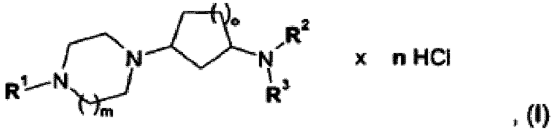 dans laquelle
m désigne le chiffre 1 ou 2,
n désigne l'un des chiffres 0, 1, 2 ou 3,
o désigne le chiffre 2,
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)-,
R² désigne
(a) H,
(b) un groupe alkyle en C₁ à C₄-O-C(O)-, benzyle-O-C(O)- et
R³ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)-
(e) acétyle, trifluoroacétyle ou trichloroacétyle,
leurs énantiomères et leurs diastéréomères, comprenant les étapes de :
(b1) réaction du composé de formule 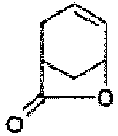 avec un composé de formule générale III 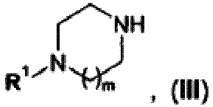 dans laquelle m et R¹ sont définis comme mentionné précédemment, en présence d'un catalyseur ;
(b2) réaction d'un composé obtenu à l'étape (b1) de formule générale X 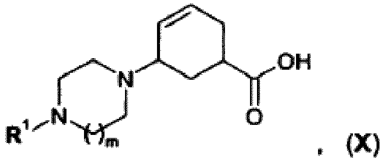 dans laquelle m et R¹ sont définis comme mentionné précédemment, en présence d'une base avec une source d'azoture, par exemple l'azoture de sodium ou l'azoture de diphénylphosphoryle (DPPA) et capture du composé formé de façon intermédiaire de formule générale XIV 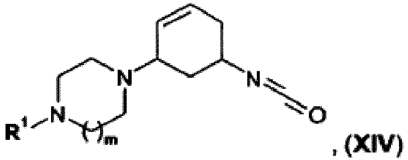 dans laquelle m et R¹ sont définis comme mentionné précédemment, avec un composé de formule générale
HO-R⁴, (XIII)
dans laquelle R⁴ représente un atome d'hydrogène, un groupe alkyle en C₁ à C₄ ou benzyle ; et
(b3) éventuellement, isolement d'un composé obtenu à l'étape (b2) de formule générale XV 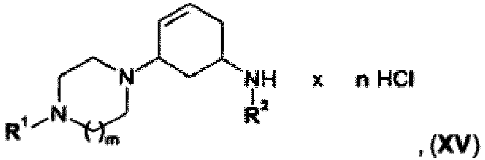 dans laquelle m, n, R¹ et R² sont définis comme mentionné précédemment ;
(b4) réduction d'un composé obtenu à l'étape (b2) ou (b3) de formule générale XV 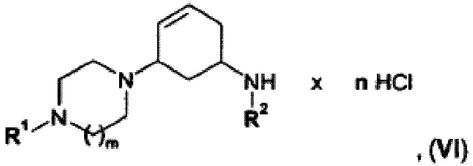 dans laquelle m et R¹ sont définis comme mentionné précédemment ; et
(b5) éventuellement, isolement d'un composé obtenu à l'étape (b4) de formule générale I 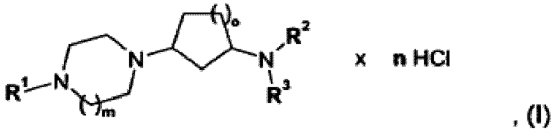 dans laquelle m, n et R¹ et R² sont définis comme mentionné précédemment.

12. Procédé selon la revendication 11, **caractérisé en ce que**
m désigne le chiffre 1,
n désigne l'un des chiffres 0, 1, 2 ou 3,
o désigne le chiffre 2,
R¹ désigne H, CH₃, un groupe benzyle, tert. butyl-O-C(O)- ou benzyle-O-C(O)-,
R² désigne H, un groupe tert. butyl-O-C(O)- ou benzyle-O-C(O)-,
R³ désigne H, CH₃, un groupe benzyle, tert. butyl-O-C(O)- ou benzyle-O-C(O)-,
leurs énantiomères et leurs diastéréomères.

13. Composés de formule générale X 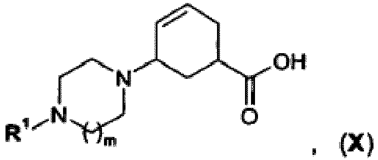 dans laquelle
m désigne l'un des chiffres 1 ou 2 et
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)-,
(e) un groupe acétyle, trichloroacétyle ou trifluoroacétyle,
leurs énantiomères et leurs diastéréomères et leurs sels et co-cristaux avec des acides chiraux ou anorganiques.

14. Composés suivants de formule générale X selon la revendication 13 :
| n° | Structure |
|---|---|
| **(1)** | 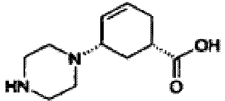 |
| **(2)** | 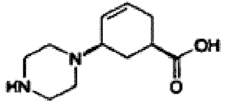 |
| **(3)** | 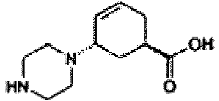 |
| **(4)** | 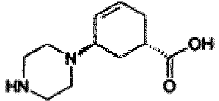 |
| **(5)** | 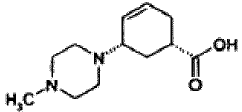 |
| **(6)** | 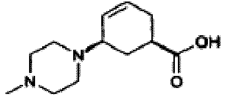 |
| **(7)** | 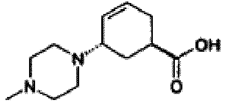 |
| **(8)** | 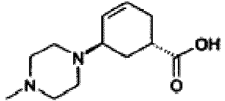 |
| **(9)** | 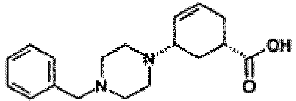 |
| **(10)** | 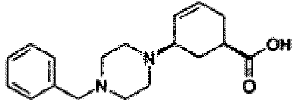 |
| **(11)** | 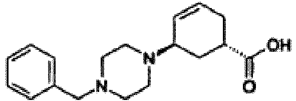 |
| **(12)** | 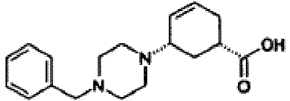 |
leurs énantiomères et leurs diastéréomères et leurs sels et co-cristaux avec des acides chiraux ou anorganiques.

15. Composés de formule générale XI 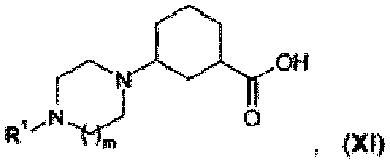 dans laquelle
m désigne l'un des chiffres 1 ou 2 et
R¹ désigne
(a) H,
(b) un groupe alkyle en C₁ à C₄, cycloalkyle en C₃ à C₆,
(c) un groupe benzyle,
(d) un groupe alkyle en C₁ à C₄-O-C(O)-, benzyle-O-C(O)-,
(e) un groupe acétyle, trichloroacétyle ou trifluoroacétyle,
leurs énantiomères et leurs diastéréomères et leurs sels et co-cristaux avec des acides chiraux ou anorganiques.

16. Composés suivants de formule générale XI selon la revendication 15 :
| n° | Structure |
|---|---|
| **(1)** | 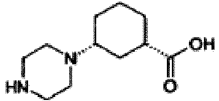 |
| **(2)** | 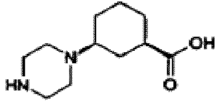 |
| **(3)** | 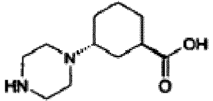 |
| **(4)** | 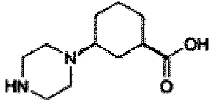 |
| **(5)** | 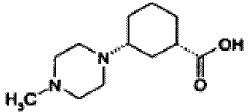 |
| **(6)** | 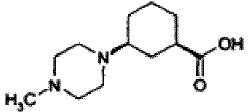 |
| **(7)** | 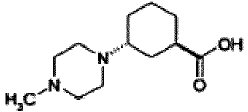 |
| **(8)** | 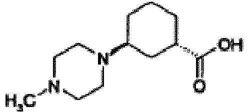 |
| **(9)** | 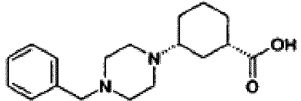 |
| **(10)** | 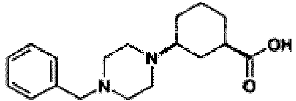 |
| **(11)** | 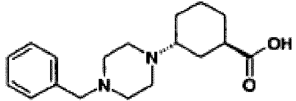 |
| **(12)** | 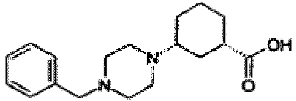 |
leurs énantiomères et leurs diastéréomères et leurs sels et co-cristaux avec des acides chiraux ou anorganiques.
